# EUROPEAN PATENT APPLICATION

(11) **EP 3 879 481 A1**
(43) Date of publication of application: **15.09.2021**
(21) Application number: 19900449.0
(22) Date of filing: 29.08.2019
(51) Int. Cl.: G06Q 40/06

(54) **CHALLENGE ASSISTANCE SYSTEM**

(30) Priority: 21.12.2018 JP 2018239548
(71) Applicant: Tanimoto, Hiroshi, Tokyo 102-0083 (JP)
(72) Inventor: Tanimoto, Hiroshi, Tokyo 102-0083 (JP)
(74) Representative: FRKelly
(86) International application number: PCT/JP2019/033926
(87) International publication number: WO 2020/129321

(57) **Abstract**

The present invention provides an attempt assistance system that increases a possibility for a user who attempts a task to achieve the task. The attempt assistance system comprises: a terminal contract generation unit that transmits, to a blockchain network, a contract condition including a condition for achieving a task and a period for attempting the task, an amount of funds to be deposited for the task, a fund distribution setting for a success of the task, and a fund distribution setting for a failure of the task; and a judgment data transmission unit that transmits, to the blockchain network, judgment data used for judging whether the contract condition has been satisfied.

## Description

### Technical Field

The present invention relates to an attempt assistance system.

### Background Art

People often have tasks they wish to achieve but have difficulty in achieving. Examples of such tasks include going for a walk to maintain or enhance one's health condition, going for a medical examination, quitting smoking, starting to learn English, and others.

In the case of these types of tasks, people other than the person who performs the task expect the person to achieve the task in order to enhance the person's health or skill. For example, the father of a person who lives an unhealthy life may wish to somehow cause his son/daughter to engage in healthy activities such as walking, or the wife or child of such person may wish to support the person to engage in healthy activities with the hope that the person will stay healthy for a long time.

### Summary

### Technical Problem

In many cases, strict deadlines are not set for such tasks, or even if a task cannot be achieved by a deadline that has been set by the task performer him/herself, there is no disadvantage that will soon arise, and tasks therefore remain permanently unachieved.

In addition, it is also difficult for other people who expect the task performer to achieve a task to continuously and proactively support the task performer despite the fact that no advantage will soon arise for themselves.

In view of the circumstances described above, an object of the present invention is to provide an attempt assistance system that increases a possibility for a user who attempts a task to achieve such task.

### Solution to Problem

An attempt assistance system according to an aspect of the present invention comprises a blockchain network, an operator server and a plurality of participant terminals, wherein the operator server comprises: a contract generation unit that transmits, to the blockchain network, a contract condition related to a task which a user should achieve, the contract condition including a condition for achieving the task and a deadline for achieving the task; an entry acceptance unit that receives information indicating an entry to the task from the participant terminal; and a participant information transmission unit that transmits, to the blockchain network, information of a participant whose entry has been accepted, and wherein the participant terminal comprises: an entry unit that transmits information indicating an entry to the task to the operator server; a setting transmission unit that transmits, to the blockchain network, an amount of funds to be deposited for the task, a fund distribution setting for a success of the task and a fund distribution setting for a failure of the task, the fund distribution setting for the success and the fund distribution setting for the failure each including one or more distributed fund receivers' information and an amount of funds to be distributed to each of the distributed fund receivers; and a judgment data transmission unit that transmits, to the blockchain network, judgment data used for judging whether the contract condition has been satisfied, wherein a smart contract in the blockchain network judges whether the contract condition has been satisfied based on the judgment data transmitted from the participant terminal and remits the funds to the respective distributed fund receivers in accordance with the fund distribution setting for the success of the task if the contract condition is satisfied by the deadline, and remits the funds to the respective distributed fund receivers in accordance with the fund distribution setting for the failure of the task if the contract condition is not satisfied by the deadline.

In the attempt assistance system, the operator server may further comprise a key generation unit that generates information for participating in the task and stores the generated information in a storage unit that is communicably connected to the operator server; and the entry acceptance unit may receive the information for participating in the task from the participant terminal, and accept an entry from the participant terminal if the information for participating in the task received from the participant terminal matches with information stored in the storage unit.

In the attempt assistance system above, the operator server may further comprise a content receiving unit that receives content and information identifying the content from the judgment data transmission unit of the participant terminal and stores the content and the information identifying the content in a storage unit which is communicably connected to the operator server, and the judgment data transmission unit of the participant terminal may transmit the judgment data including the information identifying the content to the blockchain network and transmits the content and the information identifying the content to the operator server.

In the attempt assistance system above, the attempt assistance system may further comprise a certifier terminal that comprises: a transmission unit that transmits information of a certifier to the operator server; and a judgment data transmission unit that transmits, to the blockchain network, judgment data used for judging whether the contract condition has been satisfied, wherein the operator server may further comprise a certifier registration unit that receives information of the certifier from the certifier terminal, and wherein the participant information transmission unit may transmit the information of the certifier to the blockchain network.

In the attempt assistance system above, the participant terminals may include a first participant terminal comprising at least the entry unit and the setting transmission unit, and a second participant terminal comprising at least the judgment data transmission unit.

In the attempt assistance system above, the operator server may further comprise a token contract generation unit that transmits, to the blockchain network, data related to a token for each participant, and, if the contract condition has been satisfied by the deadline, the smart contract of the blockchain network may transmit a token to a token contract generated in the blockchain network due to the transmission of the data related to the token.

A terminal device according to another aspect of the present invention comprises: an entry unit that transmits, to an operator server, information indicating an entry to a task that a user should achieve; a setting transmission unit that transmits, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers' information and an amount of funds to be distributed to each of the distributed fund receivers; and a judgment data transmission unit that transmits, to the blockchain network, judgment data used for judging whether a contract condition has been satisfied.

A method according to another aspect of the present invention comprises steps performed by a computer, the steps comprising: transmitting, to an operator server, information indicating an entry to a task that a user should achieve; transmitting, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers and an amount of funds to be distributed to each of the distributed fund receivers; and transmitting, to the blockchain network, judgment data used for judging whether a contract condition has been satisfied.

A program according to another aspect of the present invention causes a computer to perform: a process of transmitting, to an operator server, information indicating an entry to a task that a user should achieve; a process of transmitting, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers and an amount of funds to be distributed to each of the distributed fund receivers; and a process of transmitting, to the blockchain network, judgment data used for judging whether a contract condition has been satisfied.

A terminal device according to another aspect of the present invention comprises: a terminal contract generation unit that transmits, to a blockchain network, a contract condition including a condition for achieving a task and a period for attempting the task, an amount of funds to be deposited for the task, a fund distribution setting for a success of the task, and a fund distribution setting for a failure of the task; and a judgment data transmission unit that transmits, to the blockchain network, judgment data used for judging whether the contract condition has been satisfied.

In the terminal device above, a plurality of the terminal devices may include a first terminal device comprising at least the terminal contract generation unit, and a second terminal device comprising at least the judgment data transmission unit, wherein the terminal contract generation unit may further transmit, to the blockchain network, information of a person who judges whether the contract condition has been satisfied, and the second terminal device may transmit the judgment data along with information associated with the information of the person who judges whether the contract condition has been satisfied.

An attempt assistance system according to another aspect of the present invention comprises a terminal device and a server device, wherein the terminal device comprises: first transmitting unitthat transmits, to the server device, a contract condition including a condition for achieving a task and a period for attempting the task, an amount of funds to be deposited for the task, a fund distribution setting for a success of the task, and a fund distribution setting for a failure of the task; and second transmitting unitthat transmits, to the server device, judgment data used for judging whether the contract condition has been satisfied, and wherein the server device comprises: receiving unit that receives, from the terminal device, the contract condition, the amount of funds, the fund distribution setting for the success of the task, and the fund distribution setting for the failure of the task, and stores the received information in a storage unit that is communicably connected to the server device; checking unit that checks a credit of the funds from a user specified by the terminal device to a predetermined account; judging unit that judges whether the contract condition has been satisfied based on the judgment data received from the terminal device; and transferring unit that transfers the funds from the predetermined account to distributed fund receivers' account based on the fund distribution setting for the success of the task if the contract condition is satisfied during the period, while transferring the funds from the predetermined account to distributed fund receivers based on the fund distribution setting for the failure of the task if the contract condition is not satisfied during the period.

In the system above, the terminal device may comprise a first terminal device comprising at least the first transmitting unit, and a second terminal device comprising at least the second transmitting unit, wherein the first transmitting unit may further transmit, to the server device, information of a person who judges whether the contract condition has been satisfied, and the second terminal device may transmit the judgment data along with information associated with the information of the person who judges whether the contract condition has been satisfied.

An information processing device according to another aspect of the present invention comprises: first receiving unit that receives, from a first terminal device, a contract condition including a condition for achieving a task and a period for attempting the task, an amount of funds to be deposited for the task, a fund distribution setting for a success of the task, and a fund distribution setting for a failure of the task, and stores the received information in a storage unit; checking unit that checks a credit of the funds from a user specified by the first terminal device to a predetermined account; second receiving unit that receives, from a second terminal device, judgment data used for judging whether the contract condition has been satisfied; judging unit that judges whether the contract condition has been satisfied based on the judgment data; and transferring unit that transfers the funds from the predetermined account to distributed fund receivers based on the fund distribution setting for the success of the task if the contract condition is satisfied during the period, while transferring the funds from the predetermined account to distributed fund receivers based on the fund distribution setting for the failure of the task if the contract condition is not satisfied during the period.

### Advantageous Effects of Invention

According to the present invention, it is possible to provide an attempt assistance system that increases a possibility for a user who attempts a task to achieve such task.

### Brief Description of Drawings

Fig. 1 is a diagram showing a configuration example of an attempt assistance system according to an embodiment of the present invention.
Fig. 2 is a schematic diagram showing an example of information processing in an attempt assistance system according to an embodiment of the present invention.
Fig. 3 is a configuration diagram showing an operator server according to an embodiment of the present invention.
Fig. 4 is a configuration diagram showing a funds provider terminal according to an embodiment of the present invention.
Fig. 5 is a configuration diagram showing an attempter terminal according to an embodiment of the present invention.
Fig. 6 is a configuration diagram showing a certifier terminal according to an embodiment of the present invention.
Fig. 7 is a diagram showing an example of a data structure of a smart contract according to an embodiment of the present invention.
Fig. 8 is a diagram showing an example of funds provider data in the data structure of the smart contract according to an embodiment of the present invention.
Fig. 9 is a diagram showing an example of data of evidence according to an embodiment of the present invention.
Fig. 10 is a flowchart showing an entry preparation process of an attempt assistance system according to an embodiment of the present invention.
Fig. 11 is a flowchart showing entry processing of the attempt assistance system according to an embodiment of the present invention.
Fig. 12 is a flowchart showing judgment data transmission processing of the attempt assistance system according to an embodiment of the present invention.
Fig. 13 is a diagram showing an application example of the present invention.
Fig. 14 is a conceptual diagram showing a data structure of a token contract of an attempter according to an embodiment of the present invention.

### Description of Embodiments

Next, embodiments of the present invention will be described below with reference to the attached drawings. It should be noted that the following description will describe the embodiments for the purpose of facilitating the understanding of the present invention, and such embodiments are not intended to limit the interpretation of the present invention. Further, various modifications may be made to the present invention without departing from the scope of the invention. In addition, a person skilled in the art would be able to employ embodiments in which each element is substituted with its equivalent, and such embodiments are also encompassed in the scope of the present invention.

### (System Configuration)

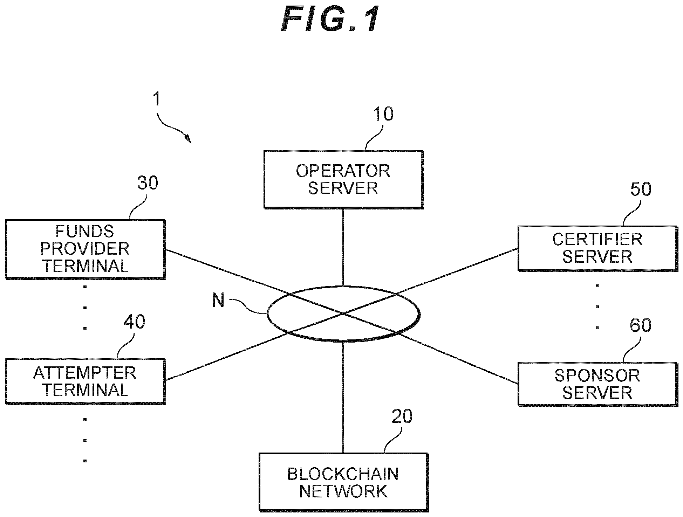

Fig. 1 is a diagram showing a configuration example of an attempt assistance system according to an embodiment of the present invention. The attempt assistance system 1 includes an operator server 10, a blockchain network 20, a funds provider terminal 30 (30a to 30L), an attempter terminal 40 (40a to 40M), a certifier terminal 50 (50a to 50N) and a sponsor server 60, and they communicate with each other through a network N. The blockchain network 20 includes a smart contract.

In the present embodiment, the blockchain network 20 is a system that regards a blockchain as a ledger and this ledger is shared and managed by a plurality of nodes. The blockchain is data that includes a plurality of blocks connected like a chain in chronological order, in which each block includes data of transactions during a certain period of time. In the blockchain technology, a plurality of blocks are added in a state in which they each hold past information and this technology is therefore characterized in that it is resistant to history falsification. Although Ethereum capable of deploying a smart contract is employed as the blockchain network 20 in the present embodiment, another type of blockchain network which is capable of deploying a smart contract may be used.

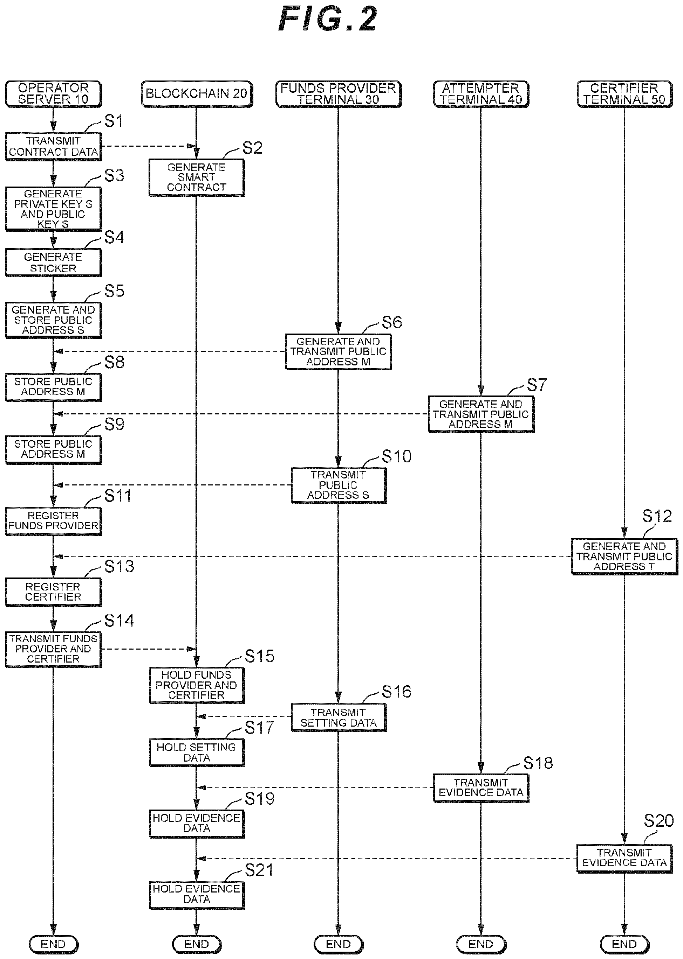

Fig. 2 is a schematic diagram showing an example of information processing in an attempt assistance system according to an embodiment of the present invention.

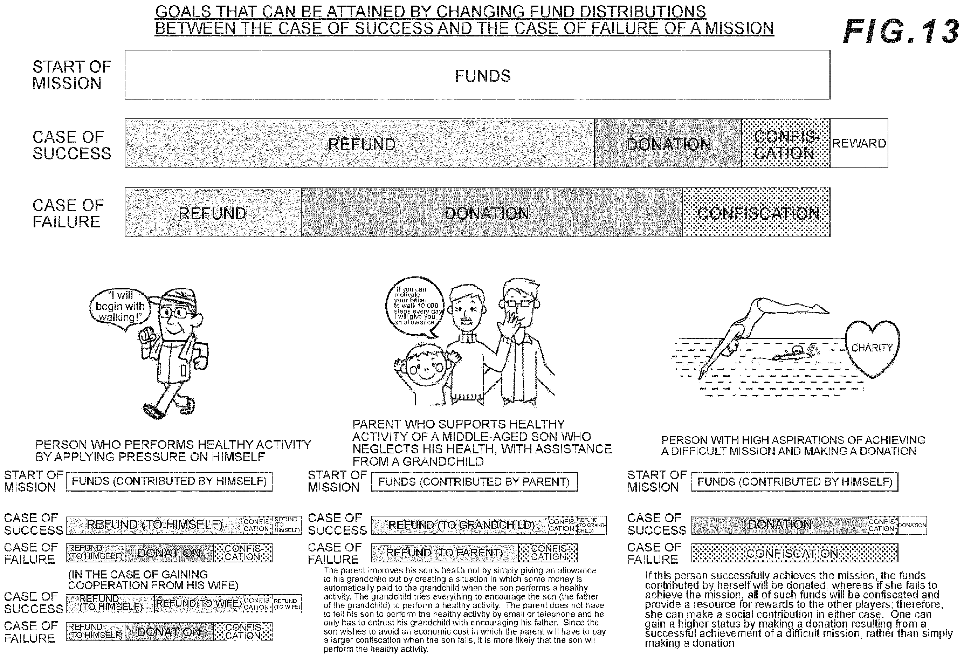

The following description assumes that an operator and a sponsor that support an attempter who is going to attempt a predetermined task have signed a contact in advance. The following description will describe an example of a sales campaign of a beverage manufacturer which supports an attempter who is going to attempt a task related to a healthy activity. The following description will describe an example in which an attempter and a funds provider are different - the attempter is a user A, the funds provider is a user B who is a father of the user A and supports a healthy activity of the user A, as shown in the center column of the bottom part of Fig. 13. A certifier is a third party who certifies the healthy activity of the attempter, which is a fitness club in the following example.

The operator and the beverage manufacturer have generated Ethereum accounts (EOA: Externally Owned Accounts) in advance and a storage unit of the operator server 10 and a storage unit of the sponsor server 60 each store a private key which was generated when the Ethereum accounts were generated and a public address generated from a public key which was generated simultaneously with the private key when the Ethereum accounts were generated. The portions in the storage unit of the operator server 10 and in the storage unit of the sponsor server 60 which store the respective private keys are usually not connected to the network and connect to the network only when necessary, which thereby enhances security.

The operator server 10 compiles, using sole, a contract code including information, such as the public address of the operator, the public address of the sponsor, the start date and time of an entry, the end date and time of the entry, the deadline for starting an attempt, an attempt period and the closing date of a smart contract, as well as the content of the contract, and transmits the compiled contract code as a transaction to the blockchain network 20 (S1). When the transaction transmitted from the operator server 10 is approved in the blockchain network 20, a smart contract is generated and its contract account is issued (S2).

The operator server 10 generates as many pairs of a public key S and a private key S for participating in the campaign of the sponsor as the number of entries to the campaign (S3), and generates a sticker on which a code obtained by encoding the private key S is printed (S4). The code records information using a contrast pattern of modules, examples of which include a barcode in which modules are arranged only in a transverse direction and a two-dimensional code in which modules are arranged in a matrix. Further, the operator server 10 stores a public address S generated using the public key S in a database (DB) included in the operator server 10 (S5) and discards the private key S. The generated stickers are delivered to the sponsor and attached onto products (e.g., drinking water) handled by the sponsor.

The user A (attempter) and the user B (funds provider) download, in advance, an attempt app 70 from the operator's website to their own terminals and use the attempt app 70 to generate a pair of a private key M and a public key M which is unique to each user. Once the public key M is generated, the attempt app 70 generates a public address M using the public key M and transmits the generated public address M to the operator server 10 (S6, S7). Upon receiving the public address M, the operator server 10 stores the public address M in the DB (S8, S9).

The user B, who supports the user A's successful task achievement, purchases a product of the beverage manufacturer which bears a sticker, and reads the code on the sticker using the attempt app 70 which has been installed in the funds provider terminal 30, whereby the public address S generated using the public key S which has been generated from the read private key S, as well as the user B's own public address M, are transmitted from the funds provider terminal 30 to the operator server 10 (S10). If the transmitted public address S matches with the public address S stored in the DB, the operator server 10 accepts an entry to the campaign of the beverage manufacturer and registers the user B's public address M as a funds provider of the campaign of the beverage manufacturer (S11). The sponsor may send a small amount of ETHER, which is a virtual currency in Ethereum, to each public address M whose entry to the campaign has been completed.

A person in charge in a fitness club (certifier) downloads, after a previous qualification by the operator, a certifier app 80 from the operator's website to the certifier terminal 50 and uses the certifier app 80 to generate a pair of a private key T and a public key T which is unique to each certifier. Once the public key T is generated, the certifier app 80 generates a public address T using the public key T and transmits the generated public address T to the operator server 10 (S12). Upon the receipt of the public address T, the operator server 10 stores the public address T in the DB (S13).

The operator server 10 transmits to the blockchain network 20, as a transaction, the public address M and the public address T, which have been registered in the DB, so as to be associated with the campaign of the beverage manufacturer (S14), to the blockchain network 20 (S14). When the transaction transmitted from the operator server 10 is approved in the blockchain network 20, the public address M and the public address T are stored in a smart contract (S15).

The user B (funds provider) transmits to the blockchain network 20 via the attempt app 70 installed in the funds provider terminal 30, as a transaction, setting data including an amount of funds to be deposited for the task, the public address M of the user A (attempter), the fund distribution setting for the success of the task, the fund distribution setting for the failure of the task, etc. (S16). Here, the fund distribution setting can select an arbitrary number of distributed fund receivers from among a plurality of distributed fund receivers who hold a public Ethereum address, such as the user A (attempter), a spouse or child of the user A, or organizations such as the Japan Red Cross Society or UNICEF, and designate an arbitrary amount of money. When the transaction transmitted from the funds provider terminal 30 is approved in the blockchain network 20, the setting data is stored in the smart contract (S17).

The user A (attempter) then transmits to the blockchain network 20, as a transaction, data serving as evidence of a healthy activity from the attempter terminal 40 (S18). When the transaction transmitted from the attempter terminal 40 is approved in the blockchain network 20, the data serving as evidence stored in the smart contract (S19). The fitness club (certifier) also transmits to the blockchain network 20, as a transaction, data serving as evidence of the user A's healthy activity from the certifier terminal 50 (S20). When the transaction transmitted from the certifier terminal 50 is approved in the blockchain network 20, the data serving as evidence is stored in the smart contract.

If the user A achieves the task by satisfying a contract condition before the closing date of the smart contract and during the attempt period, the smart contract is automatically implemented, so that the funds are distributed in accordance with the fund distribution setting for the success of the task. It should be noted that a judgment concerning time, such as the closing date of the smart contract, may be made using time stamp information of the blockchain network 20 or using time stamp information issued by a time stamping authority, which has been embedded in the transaction by a transmitter of the transaction.

If the user A cannot achieve the task before the closing date of the smart contract and during the attempt period, in a configuration in which the smart contract itself has a mechanism for obtaining information related to date and time at regular time intervals, it is determined that the task has not been successfully achieved at the point in time when the smart contract obtains the information related to date and time, and the smart contract is automatically implemented, so that the funds are distributed in accordance with the fund distribution setting for the failure of the task. If the user A cannot achieve the task before the closing date of the smart contract and during the attempt period, in a configuration in which the smart contract itself does not have a mechanism for obtaining information related to date and time at regular time intervals, the operator server, etc. transmits a transaction including date and time to the smart contract at regular time intervals, and it is determined that the task has not been successfully achieved at the point in time that such transaction is certified in the blockchain, and the smart contract is then automatically implemented, so that the funds are distributed in accordance with the fund distribution setting for the failure of the task.

The language used by the operator server 10 is not limited to the Solidity language and other languages that allow the implementation of the smart contract may be used. Further, the sources for downloading the attempt app 70 and the certifier app 80 are not limited to the operator's website, and attempters and certifiers may download the apps from websites operated by others.

### (Configuration of Operator Server)

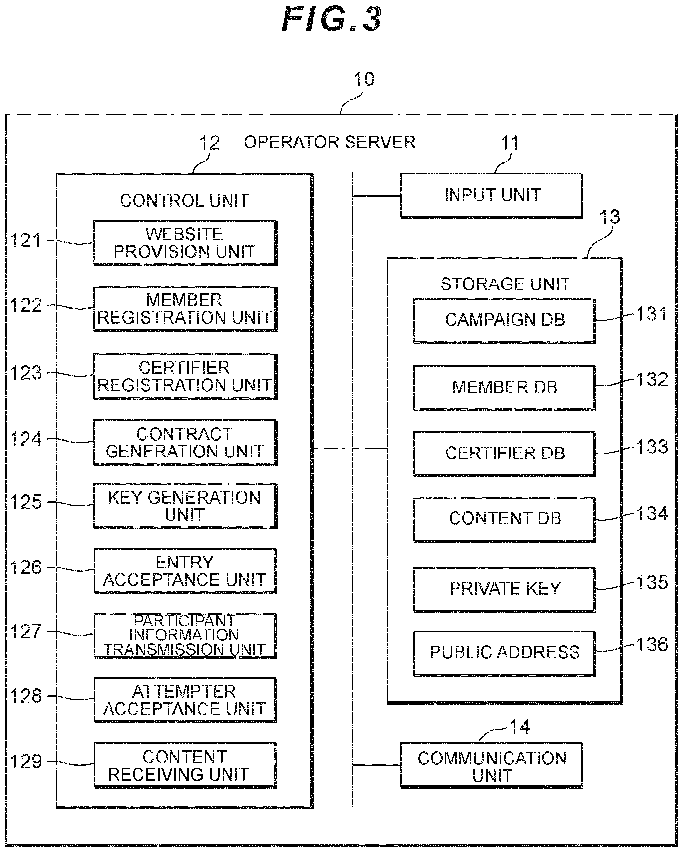

Fig. 3 is a configuration diagram showing the operator server 10 according to an embodiment of the present invention. Fig. 3 assumes a single operator server 10 and only shows necessary functional configurations; however, the operator server 10 may be configured as part of a multifunctional distributed system consisting of a plurality of computer systems.

The operator server 10 is a server device having a function of communicating with the blockchain network 20, the funds provider terminal 30, the attempter terminal 40, the certifier terminal 50 and the sponsor server 60 via the network N. As shown in Fig. 3, the operator server 10 includes an input unit 11, a control unit 12 including a CPU and a memory, a communication unit 14 for connecting the storage unit 13 and the network N to each other.

When programs stored in storage unit 13 are read out by RAM and executed by the CPU, the control unit 12 functions as a website provision unit 121, a member registration unit 122, a certifier registration unit 123, a contract generation unit 124, a key generation unit 125, an entry acceptance unit 126, a participant information transmission unit 127, an attempter acceptance unit 128 and a content receiving unit 129. It is preferable that the storage unit 13 has a campaign DB 131, a member DB 132, a certifier DB 133, a content DB 134, a private key 135 and a public address 136.

The campaign DB 131 stores information related to a campaign. In an embodiment, the campaign DB 131 has a plurality of public addresses S registered thereto so as to be associated with campaign IDs and contract accounts. The campaign ID is an ID that uniquely identifies each campaign. The "ID" herein refers to information consisting of one or more *kanji* characters, *hiragana* characters, *katakana* characters, alphanumeric characters, symbols and the like. The contract account is information that uniquely identifies each smart contract generated in the blockchain network 20. The public address S is information generated using the public key S in the pair of a private key and a public key generated for the campaign.

The member DB 132 stores information related to members of the attempt assistance system 1. In one embodiment, the member DB 132 preferably has a name, a gender, a date of birth, a public address M, a relevant campaign, etc. registered thereto. The public address M is information that uniquely identifies each member in the attempt assistance system 1. The relevant campaign includes a campaign ID with which the user of the public address M is associated, and a participation status with respect to the campaign. The participation status may include, for example, a "funds provider" indicating that the participant is a funds provider of the campaign, a "attempter" indicating that the participant is an attempter of the campaign, and so on. It should be noted that the relevant campaign may store information related to an arbitrary campaign with which the user of the public address M is associated.

The certifier DB 133 stores information related to certifiers of the attempt assistance system 1. In one embodiment, the certifier DB 133 preferably has a certifier's name, a certifier's qualification, a location, a public address T, a designated campaign, etc. registered thereto. The public address T is information that uniquely identifies each certifier of the attempt assistance system 1. The designated campaign includes information related to a campaign for which the certifier has been qualified by the operator.

In one embodiment, when a specific campaign has been qualified by the operator, the designated campaign includes a campaign ID of the qualified specific campaign, whereas, when all campaigns have been qualified by the operator, the designated campaign includes "ALL" which indicates that all of the campaigns have been qualified. In one embodiment, after the qualification by the operator, a user serving as the operator may store data in the designated campaign.

The content DB 134 stores information related to content, such as an image and a sound, which serves as evidence of an activity for achieving a task in the attempt assistance system 1. In one embodiment, the content DB 134 preferably has a content address and content registered thereto. The content address is information that uniquely identifies each content item. In one embodiment, a hash value generated from image data, etc. may be used as the content address.

The private key 135 is a private key generated when an Ethereum account is generated. It is preferable that the private key 135 is normally stored off-line and accessible when needed.

The public address 136 is a public address that is generated from a public key generated at the same time as a private key which is issued when an Ethereum account is generated.

The website provision unit 121 provides, in the network N, a website for utilizing the attempt assistance system 1. In this website, funds providers, attempters and refunds receivers who will receive a refund can register themselves as members, certifiers can register themselves as certifiers, and users who have been registered as members or certifiers can download the attempt app 70 or the certifier app 80. For such purpose, the website provision unit 121 transmits a webpage described in HTML (HyperText Markup Language), etc. to terminals used by users and receives user input results from the user terminals.

The member registration unit 122 receives member information from a user terminal and registers the member information in the member DB 132. In the present embodiment, when member information is transmitted from a terminal in response to a user's operation in a member registration screen provided by the website provision unit 121, the member registration unit 122 registers a record in the member DB 132 based on the received member information. The member information includes a name, a gender, a date of birth, and so on.

Upon receipt of a public address M along with user identification information from a terminal to which the attempt app 70 has been downloaded, the member registration unit 122 stores the public address M in the record in the member DB 132 which is specified by the user identification information. The user identification information may be information generated by using part or all of the member information which is obtained by, for example, prompting the user to input when the attempt app 70 is installed or started.

The certifier registration unit 123 receives certifier information from the certifier terminal 50 and registers the received certifier information to the certifier DB 133. In the present embodiment, when the certifier information is transmitted from the certifier terminal 50 in response to the user's operation in a certifier registration screen provided by the website provision unit 121, the certifier registration unit 123 registers a record to the certifier DB 133 based on the received certifier information. The certifier information includes a certifier's name, a certifier's qualification, a location, and so on.

Upon receipt of a public address T along with certifier identification information from the certifier terminal 50 to which the certifier app 80 has been downloaded, the certifier registration unit 123 stores the public address T in the record in the certifier DB 133 which is specified by the certifier identification information. The certifier identification information may be information generated by using part or all of the certifier information which is obtained by, for example, prompting the certifier to input when the certifier app 80 is installed or started.

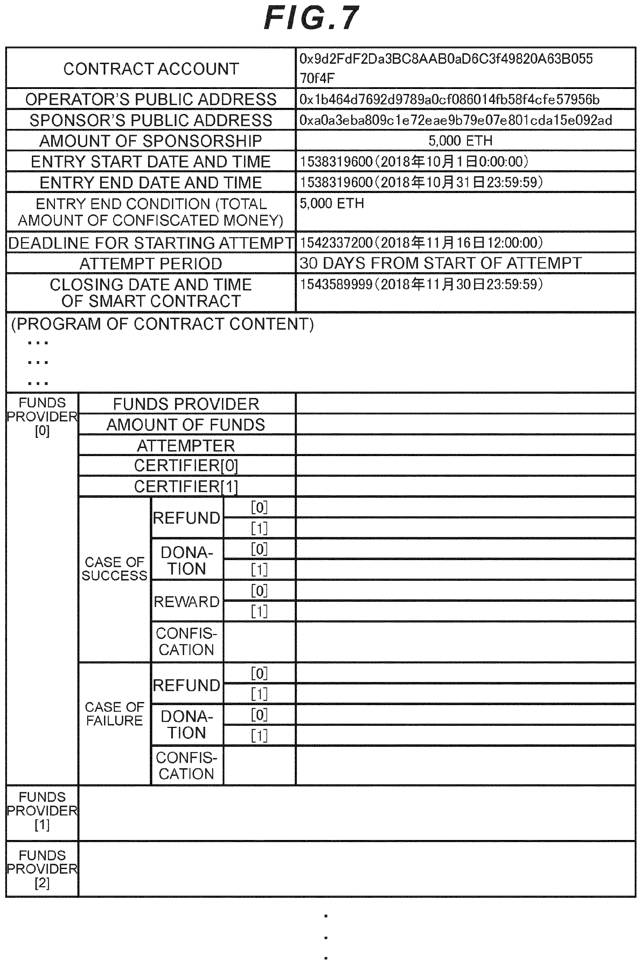

The contract generation unit 124 compiles, using sole, a contract code describing information, such as a public address of the operator, a public address of the sponsor, an entry start date and time, an entry end date and time, a deadline for starting the attempt, an attempt period, the closing date of the smart contract, as well as the content of the contract, which are described using the Solidity language, and transmits the compiled contract code as a transaction to the blockchain network 20, whereby a smart contract is generated. The contract generation unit 124 stores a contract account issued when the smart contract is generated in the campaign DB 131. In the present embodiment, the contract generation unit 124 generates a smart contract as shown in Fig. 7.

In the present embodiment, as shown in Fig. 7, the generated smart contract includes as many funds provider data sequences as the number of entries to the campaign, the funds provider data sequences being intended to store various types of setting data from the respective funds providers. For example, in the present embodiment, the funds provider data includes a funds provider, an amount of funds, an attempter, a certifier, a fund distribution setting for the success of a task, and a fund distribution setting for the failure of the task, and the values of these items are each null at the time of generation of the smart contract. Data is stored in each of these items of the funds provider data by processes performed by the participant information transmission unit 127 of the operator server 10 and a setting transmission unit 334 of the funds provider terminal 20 which will be described later.

Each fund distribution setting includes refund, donation and confiscation, and the funds provider terminal 30 transmits a fund distribution setting which specifies the amounts of money for the refund, the donation and the confiscation such that the sum thereof matches with the amount of funds.
As the refund, an arbitrary refund receiver who holds a public address of the Ethereum and an arbitrary amount of money may be designated. As the donation, an arbitrary donation receiver who holds a public address of the Ethereum and an arbitrary amount of money may be designated. As the confiscation for a case of success, although an operator fee to be paid to the operator is typically set, an arbitrary amount of money equal to or greater than the operator fee may arbitrarily be designated. As the confiscation for a case of failure, an arbitrary amount of money equal to or greater than the operator fee may be designated. The fund distribution setting for the success of a task may include a reward. As the reward, a receiver of funds to be distributed as a reward when the task is successfully achieved may be designated.

The key generation unit 125 generates as many pairs of a private key S and a public key S as the number of entries to the campaign, each pair being used for verifying a user to be entered to the campaign of the sponsor. The key generation unit 125 generates a public address S using the public key S and stores the public address S in the campaign DB 131 so as to be associated with a campaign ID. The campaign ID may be generated by the key generation unit 125 or may be generated in advance by another functional unit in the operator server 10.

In one embodiment, the key generation unit 125 generates a code obtained by encoding the private key S and outputs the encoded code. Although the operator generates a sticker with the encoded code printed thereon and delivers the sticker to the sponsor in the present embodiment, the sponsor who has obtained the data of the encoded code may alternatively generate a sticker with the encoded code printed thereon in another embodiment.

The entry acceptance unit 126 receives, from the funds provider terminal 30, the public address S and the public address M of the user of the funds provider terminal 30 and judges whether the received public address S matches with the public address S stored in the campaign DB 131. When matched, the entry acceptance unit 126 stores, in a record having the corresponding public address M in the member DB 132, the campaign ID associated with the public address S as a relevant campaign, and a participation status "funds provider." When the received public address S matches with the public address S stored in the campaign DB 131, the entry acceptance unit 126 may render the public address S stored in the campaign DB131 non-reusable as it is "used" or may delete it , in order to avoid redundant use of the public address S.

The participant information transmission unit 127 transmits to the blockchain network 20, as a transaction, the public address M and the public address T, which have been registered to the DB so as to be associated with the campaign, so that the public address M and the public address T are stored in the smart contract of the campaign. The participant information transmission unit 127 may transmit, in response to the entry acceptance unit 126 receiving the public address M, the received public address M and the public address T as a transaction, or may collectively transmit public addresses M, which have been received in a certain period of time, along with the public addresses T as a transaction at predetermined regular intervals. As a result, the transmitted public address M is stored as the "funds provider" of the funds provider data in the smart contract and the public address T which has been transmitted along with the public address M is stored as the "certifier."

The attempter acceptance unit 128 receives, from the funds provider terminal 30, information related to the campaign and the public address M of the attempter and stores, in a record having the corresponding public address M in the member DB 132, the campaign ID specified by the information related to the campaign as the relevant campaign and a participation status as "attempter." In the present embodiment, the attempter acceptance unit 128 receives the contract account and the public address M from the funds provider terminal 30 and stores, in a record having the received public address M, the campaign ID specified by the contract account and the participation status as "attempter."

The content receiving unit 129 receives content such as image data and sound data from the attempter terminal 40 or the certifier terminal 50 and stores the received content in the content DB 134. In the present embodiment, the content receiving unit 129 receives content and content address that uniquely identifies each content item, and stores the received content address and the received content in the content DB 134. The content may be data, such as images and sounds, which serve as evidence of an activity for achieving a task. Examples of the images may include a meal photo captured by the attempter, a body measurement image captured by the attempter, and an inspection image captured by the certifier.

### (Configuration of Funds provider Terminal)

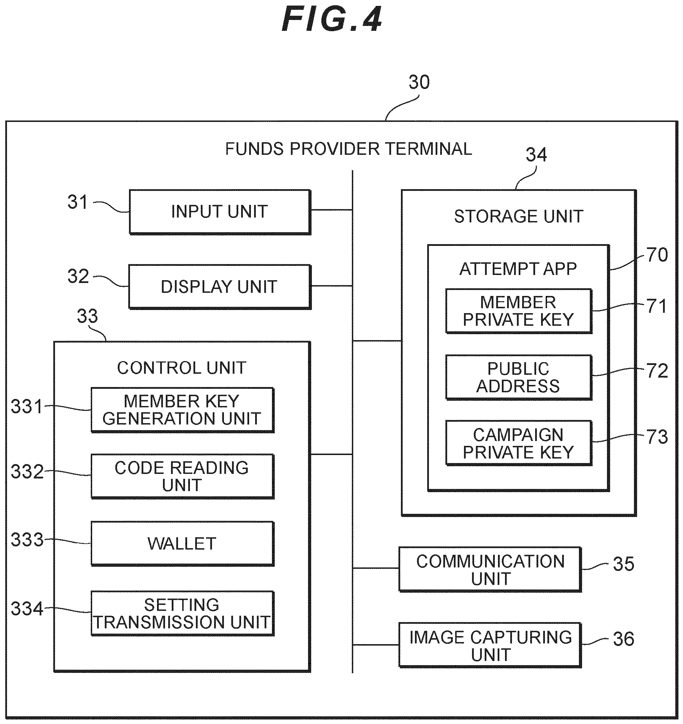

Fig. 4 is a configuration diagram showing the funds provider terminal 30 according to an embodiment of the present invention. The funds provider terminal 30 is an information processing terminal having a function of communicating with the operator server 10 and the blockchain network 20 via the network N. Specifically, examples of the funds provider terminal 30 may include, without limitation, a portable phone or a smartphone, a PC, a PDA and a tablet.

As shown in Fig. 4, the funds provider terminal 30 includes an input unit 31, such as a touch panel, which is operated by a user, a display unit 32 such as a display, a control unit 33 that includes a CPU and a memory, a storage unit 34, a communication unit 35 for connecting the funds provider terminal 30 to the network N, an image capturing unit 36 and so on. In the present embodiment, the storage unit 34 stores the attempt app 70. The attempt app 70 preferably has a member private key 71, a public address 72 and a campaign private key 73.

When the attempt app 70 stored in the storage unit 34 is read out by RAM and executed by the CPU, the control unit 33 functions as a member key generation unit 331, a code reading unit 332, a wallet 333 and a setting transmission unit 334.

The member key generation unit 331 generates a pair of a private key M and a public key M which is unique to each user who has been registered as a member, and stores the generated private key M as a member private key 71 of the storage unit 34. It is preferable that the member private key 71 is generated off-line, normally stored off-line and accessible when needed. The member key generation unit 331 generates a public address M using the public key M and stores the generated public address M in the public address 72 of the storage unit 34. Further, the member key generation unit 331 transmits the generated public address M along with the user identification information to the operator server 10. As stated earlier, the user identification information may be information generated by using part or all of the member information which is obtained by, for example, prompting the user to input when the attempt app 70 is installed or started.

The code reading unit 332 reads a private key S from an image including a code, which has been obtained by the image capturing unit 36, and stores the read private key S as the campaign private key 73. The code reading unit 332 generates a public key S from the read private key S, generates a public address S using the public key S, and transmits the public address S and its own public address M to the operator server 10.

The wallet 333 has a function of managing a virtual currency. In the present embodiment, the wallet 333 transmits to the blockchain system 20, as a transaction, fund payment data including an account of a beneficiary and an amount of remittance. ample, the wallet 333 may sign the fund payment data including a contract account designated by the user via the input unit 31 and the amount of remittance with the member private key 71 in the storage unit 34, and transmit the signed fund payment data as a transaction to the blockchain system 20. When the transaction is approved by the blockchain network 20, the funds sent to the smart contract are stored. The wallet 333 may obtain a list of contract accounts from the operator server 10 and display the list in a form selectable by the user. When the contract accounts are obtained, the wallet 333 may transmit the public address 72 of the storage unit 34, so that the list of contract accounts associated with the campaigns for which the user has been registered to the operator server 10 as a funds provider can be obtained.

The setting transmission unit 334 transmits data including various types of setting data as a transaction to the blockchain system 20. In the present embodiment, the setting data includes the public address M of the funds provider, the amount of funds, the public address M of the attempter, the fund distribution setting for the success of the task, the fund distribution setting for the failure of the task, etc. For example, the setting transmission unit 334 may sign the various types of setting data designated by the user via the input unit 31 signed by the member private key 71 in the storage unit 34 and transmit the signed setting data as a transaction to the blockchain network 20. When the transaction is approved by the blockchain network 20, the various types of setting data are stored in the smart contract. The setting transmission unit 334 may obtain a list of contract accounts from the operator server 10 and display the list in a form selectable by the user. When the contract accounts are obtained, the setting transmission unit 334 may transmit the public address 72 of the storage unit 34, so that the list of contract accounts associated with the campaigns for which the user has been registered to the operator server 10 as a funds provider can be obtained.

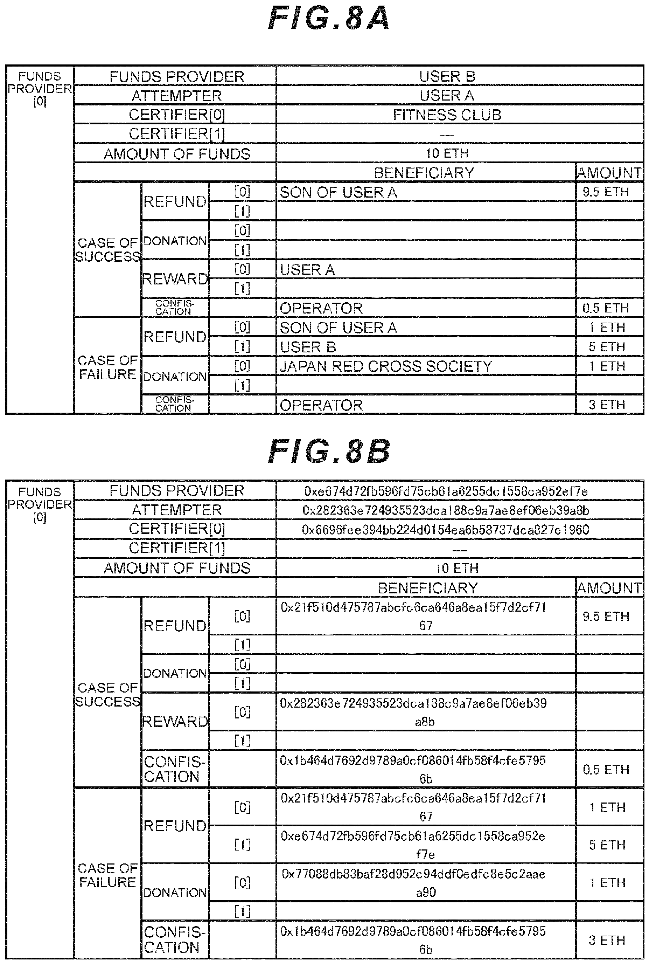

As a result of the processes stated above, a value is stored for each of the items of the funds provider data that stores the public address M of the corresponding funds provider as shown in Fig. 8. Fig. 8(A) shows the content of data set by the attempt app 70 of the funds provider terminal 30 and Fig. 8(B) shows the data structure of a smart contract stored by the transaction transmitted based on the data shown in Fig. 8(A).

The setting transmission unit 334 transmits the address M of the attempter designated in the setting data, along with the information of a campaign to be attempted, to the operator server 10. In the present embodiment, the setting transmission unit 334 transmits the public address M along with the contract account to the operator server 10.

### (Configuration of Attempter Terminal)

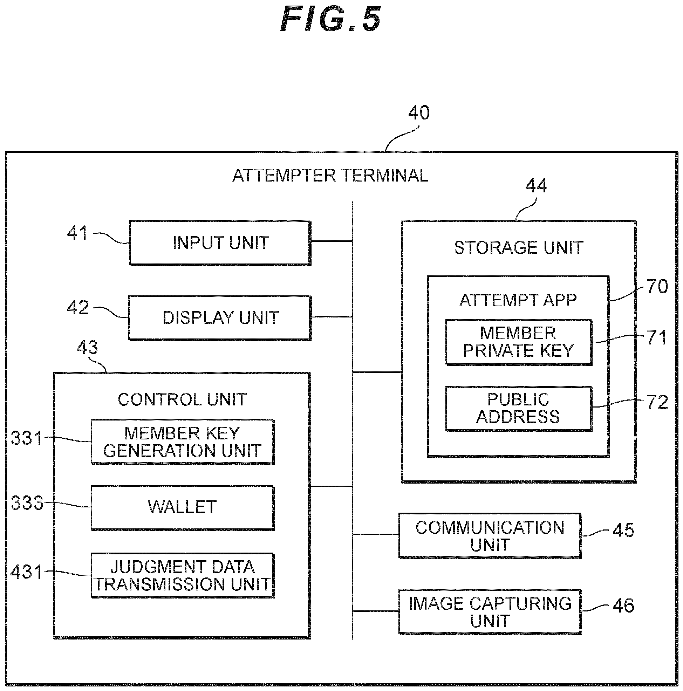

Fig. 5 is a configuration diagram showing the attempter terminal 40 according to an embodiment of the present invention. The attempter terminal 40 is an information processing terminal having a function of communicating with the operator server 10 and the blockchain network 20 via the network N. Specifically, examples of the attempter terminal 40 may include, without limitation, a portable phone or a smartphone, a PC, a PDA and a tablet.

As shown in Fig. 5, the attempter terminal 40 includes an input unit 41 such as a touch panel which is operated by a user, a display unit 42 such as a display, a control unit 43 that includes a CPU and a memory, a storage unit 44, a communication unit 45 for connecting the attempter terminal 40 to the network N, an image capturing unit 46 and so on. In the present embodiment, the storage unit 44 stores the attempt app 70. The attempt app 70 preferably has a member private key 71 and a public address 72.

When the attempt app 70 stored in the storage unit 44 is read out by RAM and executed by the CPU, the control unit 43 functions as a member key generation unit 331, a wallet 333 and a judgment data transmission unit 431.

Since the member key generation unit 331 and the wallet 333 have already been described in the section related to the configuration of the funds provider terminal, their description thereof will be omitted in the following description.

The judgment data transmission unit 431 transmits to the blockchain network 20, as a transaction, judgment data used for judging whether a contract condition has been satisfied. In the present embodiment, the judgment data transmission unit 431 signs the judgment data with the member private key 71 in the storage unit 44 and transmits the signed judgment data to the blockchain network 20. Arbitrary types of data may be employed as the judgment data, which may include data that serves as evidence of an activity for achieving a task, data including either "success" or "failure" of the task, etc.

The judgment data transmission unit 431 may obtain a list of contract accounts from the operator server 10 and display the list in a form that allows the user to select a destination of the transaction. When the contract accounts are obtained, the judgment data transmission unit 431 may transmit the public address 72 of the storage unit 44, so that the list of contract accounts of the campaigns for which the user has been registered to the operator server 10 as an attempter can be obtained.

Regarding content in judgment data, the judgment data transmission unit 431 generates a content address that uniquely identifies each content item, transmits the generated content address as a transaction to the blockchain network 20, and transmits actual content along with the content address to the operator server 10. As stated earlier, examples of images may include a meal image captured by the attempter, a body measurement image captured by the attempter, an inspection image captured by the certifier, and the like.

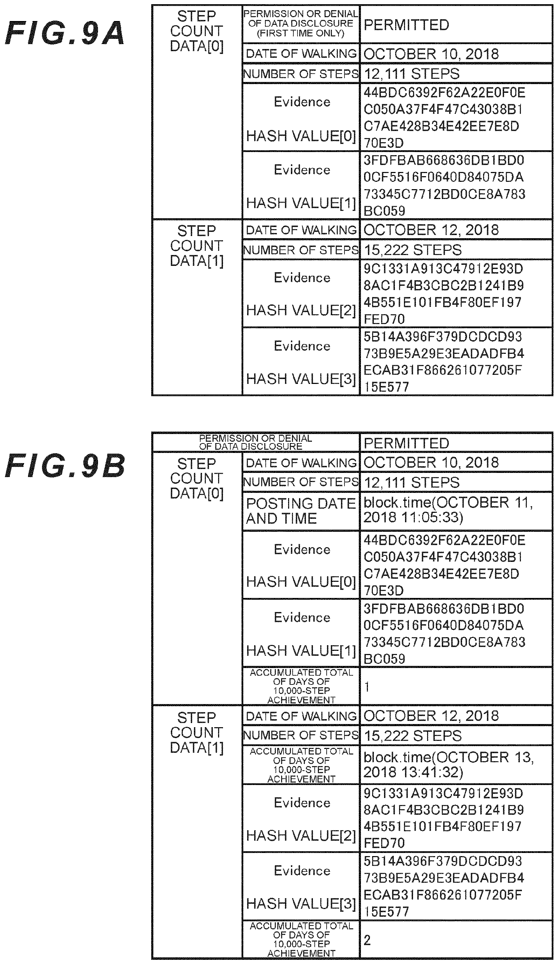

Fig. 9 is a diagram showing an example of data related to evidence according to an embodiment of the present invention. Fig. 9(A) shows data transmitted from the attempter terminal 40 to the blockchain network 20, and Fig. 9(B) shows a data structure of a smart contract stored by the transmitted transaction. Fig. 9 shows an example of data that serves as evidence of walking for achieving a task of walking 15 days in total during 30 days from the start of the attempt and up to a predetermined deadline, each day with 10,000 or more steps.

### (Configuration of Certifier Terminal)

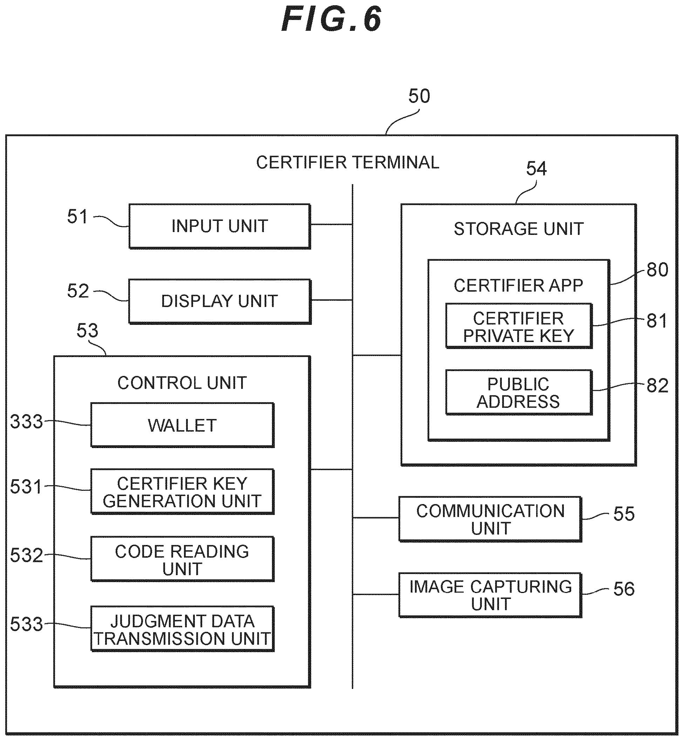

Fig. 6 shows a configuration of the certifier terminal 50 according to an embodiment of the present invention. The certifier terminal 50 is an information processing terminal having a function of communicating with the operator server 10 and the blockchain network 20 via the network N. Specifically, examples of the certifier terminal 50 may include, without limitation, a portable phone or a smartphone, a PC, a PDA and a tablet.

As shown in Fig. 6, the certifier terminal 50 includes an input unit 51 such as a touch panel which is operated by a user, a display unit 52 such as a display, a control unit 53 that includes a CPU and a memory, a storage unit 54, a communication unit 55 for connecting the certifier terminal 50 to the network N, an image capturing unit 56, and so on. In the present embodiment, the storage unit 54 stores the certifier app 80. The certifier app 80 preferably has a certifier private key 81 and a public address 82.

When the certifier app 80 stored in the storage unit 54 is read out by RAM and executed by the CPU, the control unit 53 functions as a wallet 333, a certifier key generation unit 551, a code reading unit 532 and a judgment data transmission unit 533.

Since the wallet 333 has already been described in the section related to the configuration of the funds provider terminal, the description thereof will be omitted in the following description.

The certifier key generation unit 531 generates a pair of a private key T and a public key T that is unique to each certifier who has been registered as a certifier, and stores the generated private key T as the certifier private key 81 of the storage unit 54. The certifier private key 81 is preferably generated off-line, normally stored off-line, and accessible when needed. The certifier key generation unit 531 generates a public address T using the public key T and transmits the generated public address T along with certifier identification information to the operator server 10. As stated earlier, the certifier identification information may be information generated by using part or all of the certifier information which has been obtained by, for example, prompting the certifier to input when the certifier app 80 is installed or started.

The code reading unit 532 reads a public address M from an image including a code, which has been obtained by the image capturing unit 56.

The judgment data transmission unit 533 has a similar function to the judgment data transmission unit 431 of the attempter terminal 40 and transmits to the blockchain network 20, as a transaction, data that serves as evidence of an activity for achieving a task. In the present embodiment, the judgment data transmission unit 533 generates data that serves as evidence based on the public address M read by the code reading unit 532, signs the data with the certifier private key 81 of the storage unit 54 and transmits the signed data to the blockchain network 20.

The judgment data transmission unit 533 may obtain a list of contract accounts from the operator server 10 and display the list in a form that allows the user to select a destination of the transaction. When the contract accounts are obtained, the judgment data transmission unit 533 may transmit the public address 82 of the storage unit 54, so that the list of contract accounts of the campaigns which have been registered to the operator server 10 as the campaigns designated by the certifier.

Regarding the content in the data that serves as evidence, the judgment data transmission unit 533 generates a content address that uniquely identifies each content item, transmits the generated content address as a transaction to the blockchain network 20, and transmits actual content along with the content address to the operator server 10.

### (Configuration of Sponsor Server)

The sponsor server 60 is a server device having a function of communicating with the operator server 10 and the blockchain network 20 via the network N. Although Fig. 1 shows a single sponsor server 60 for a simple explanation, there may be a plurality of sponsor servers 60 in the case of a plurality of sponsors gathering together for one sales campaign and, additionally or alternatively, there may be different sponsor servers 60 related to different sales campaigns.

### (First Embodiment)

A first embodiment of the present invention will describe an example of a sales campaign of a beverage manufacturer which supports an attempter who attempts a task related to healthy activities. The following description assumes that a beverage manufacture, who serves as a sponsor, has signed a contract in advance with an operator, and the beverage manufacturer has paid an agent service fee of X yen and support money of 5000 ETHER to the operator. In the present embodiment, if an attempter walks 15 days in total during a 30-day period, each day with 10,000 or more steps, the task is considered to have been successfully achieved. The following description will describe an example in which an attempter and a funds provider are different, wherein the attempter is a user A and the funds provider is a user B who is a father of the user A and who supports the healthy activities of the user A. The certifier is a fitness club who certifies the healthy activities of the attempter.

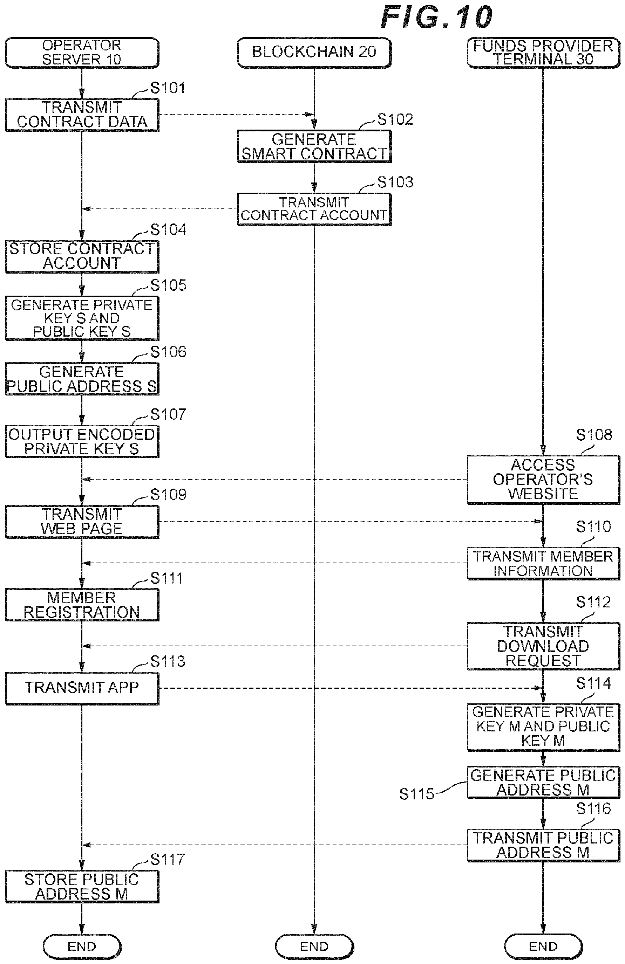

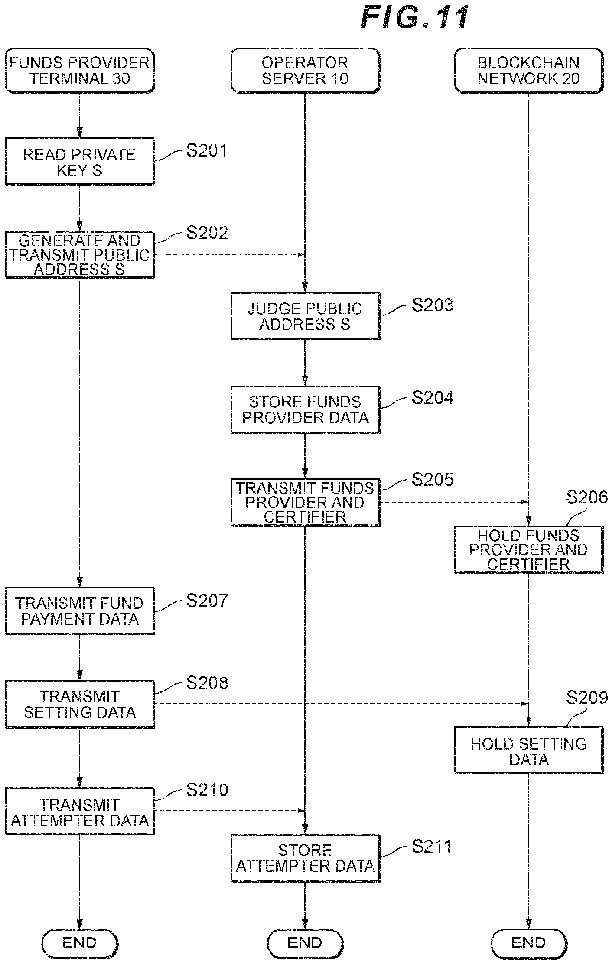

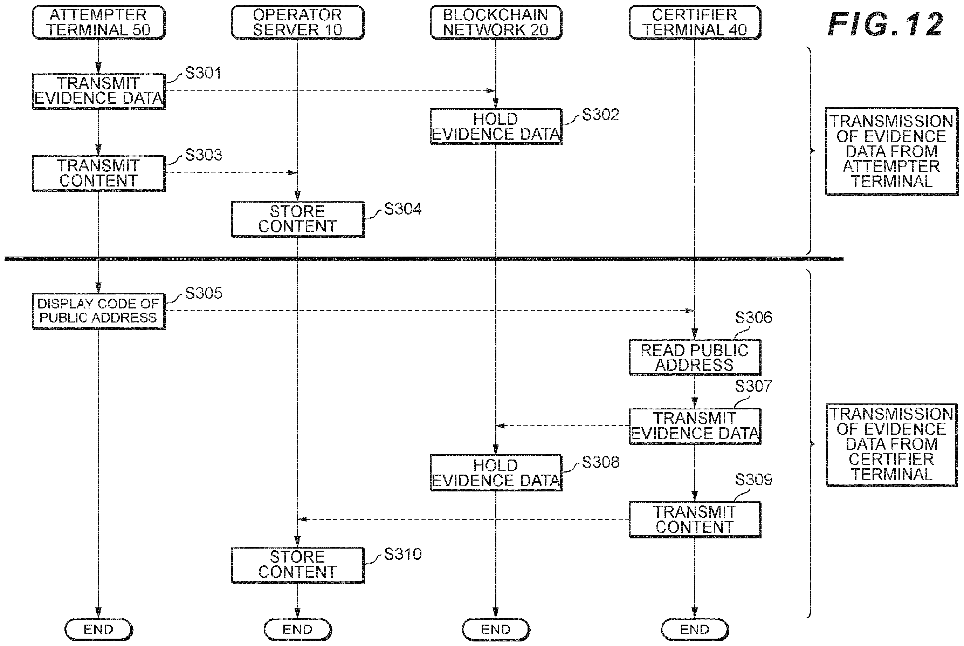

The operator and the beverage manufacturer have each generated an Ethereum account in advance, and the storage unit 13 of the operator server 10 and the storage unit of the sponsor server 60 have each stored: a private key generated when the Ethereum account was issued; and a public address generated from a public key which was generated when the Ethereum account was issued. First, entry preparation processing will be described with reference to Fig. 10, then entry processing will be described with reference to Fig. 11, and lastly, judgment data transmission processing will be described with reference to Fig. 12.

### (Entry Preparation Processing)

The contract generation unit 124 of the operator server 10 compiles, using sole, a contract code including information, such as the public address of the operator, the public address of the sponsor, an entry start date and time, an entry end date and time, a deadline for starting the attempt, an attempt period, the closing date of the smart contract, as well as the content of the contract, which are described using the Solidity language, and transmits the compiled contract code to the blockchain network 20 (S101) as a transaction. When the transaction transmitted from the operator server 10 is approved in the blockchain network 20, a smart contract is generated (S102).

In the present embodiment, the smart contract as shown in Fig. 7 is generated as a result of the transmission of the transaction from the contract generation unit 124. The content of the smart contract is as follows.
(1) During a time period from the entry start date and time to the entry end date and time, the funds provider transmits, as a transaction, data that includes various types of setting data (a public address M of the funds provider, the amount of funds, a public address M of the attempter, the fund distribution setting for the success of the task, the fund distribution setting for the failure of the task, etc.) and fund payment data, whereby an entry is accepted.
(2) By the deadline for starting the attempt, the attempter transmits to the blockchain network 20, as a transaction, data that serves as evidence of an activity for achieving the task, whereby the attempt of the attempter starts.
(3) During the attempt period, if the attempter performs a healthy activity in a store, etc. of the certifier, the certifier transmits to the blockchain network 20, as a transaction, the data that serves as evidence of the healthy activity performed by the attempter.
(4) When the accumulated total number of days in each of which the attempter walks 10,000 or more steps becomes 15 days during the attempt period, the task is successfully achieved.
(5) If the attempter achieves the task before the closing date of the smart contract, or if the attempter cannot achieve the task before the closing date of the smart contract and during the attempt period, the operator fee is automatically remitted to the operator.
(6) If the attempter achieves the task before the closing date of the smart contract and during the attempt period, or if the attempter cannot achieve the task before the closing date of the smart contract and during the attempt period, compensation is automatically remitted to the certifier.
(7) If the attempter achieves the task before the closing date of the smart contract, a refund is automatically remitted to a refund receiver and/or a donation is automatically remitted to a donation receiver, in accordance with the fund distribution setting for the success of the task.
(8) If the attempter cannot achieve the task before the closing date of the smart contract and during the attempt period, a refund is automatically remitted to a refund receiver and/or a donation is automatically remitted to a donation receiver, in accordance with the fund distribution setting for the failure of the task.
(9) An amount of money obtained by subtracting, from an amount of confiscation, the operator fee for the operator plus an additional amount (α) and the compensation for the certifier, is remitted as a reward for the successful achievement to the public addresses designated in the respective smart contracts such that the amounts of money remitted to the respective smart contracts which have achieved the task become equal.
(10) For a challenger who has agreed to disclose various data including content related to the challengers own evidence to the sponsor and achieved the task before the closing date of the smart contract and during the challenge period, a predetermined amount of money from sponsor money is automatically remitted to the challenger and/or the refund receiver.
(11) An access key for accessing data of the attempter who has agreed to disclose various data including content related to the attempter's own evidence, as well as a content address of the content, are automatically transmitted to the sponsor.
(12) The sponsor can access the attempter's data which has been encrypted and stored in the operator server using the access key. In the operator server, the attempter's content is managed using the content address.

In the present embodiment, the generated smart contract includes as many funds provider data sequences as the number of entries to the campaign, the funds provider data sequences being intended to store various types of data settings from the respective funds providers, as shown in Fig. 7. For example, in the present embodiment, the funds provider data includes a funds provider, an amount of funds, an attempter, a certifier, a fund distribution setting for the success of a task, and a fund distribution setting for the failure of the task.

Each fund distribution setting includes a refund, donation and confiscation, and the funds provider designates an amount of money for each of the refund, the donation and the confiscation such that the sum thereof matches with the amount of funds. As the refund, an arbitrary refund receiver who holds a public Ethereum address and an arbitrary amount of money may be designated. As the donation, an arbitrary donation receiver who holds a public Ethereum address and an arbitrary amount of money may be designated. As the confiscation for a successful case, an operator fee to be paid to the operator is set. As the confiscation for a failure case, an arbitrary amount of money equal to or greater than the operator fee may be designated. The fund distribution setting for the success of a task may include a reward. As the reward, a receiver of funds to be distributed as a reward when the task is successfully achieved may be designated.

Referring back to Fig. 10, the contract generation unit 124 receives, from the blockchain network 20, the contract account issued when the smart contract is generated (S103), and stores the contract account in the campaign DB 131 (S104). The present embodiment assumes that the contract generation unit 124 generates a campaign ID "001," and stores the received contract account in the campaign DB 131 so as to be associated with the campaign ID "001."

The key generation unit 125 of the operator server 10 generates as many pairs of a private key S and a public key S as the number of entries to the campaign, each pair being used for verifying a user to be entered to the campaign of the beverage manufacturer (S105). The key generation unit 125 also generates a public address S using the public key S and stores the public address S in the campaign DB 131 so as to be associated with a campaign ID (S106). The present embodiment assumes that the key generation unit 125 stores 1,000 public addresses S in the campaign DB 131 so as to be associated with the campaign ID "001."

The key generation unit 125 further generates a code obtained by encoding the private key S and outputs the encoded code (S107). In the present embodiment, the operator generates a sticker with the encoded code printed thereon and delivers the sticker to the sponsor.

When the funds provider accesses, using a browser of the funds provider terminal 30, a website provided by the operator server 10 (S108), the website provision unit 121 of the operator server 10 transmits a web page according to the accessed URL to the funds provider terminal 30 (S109).

When member information required for member registration is transmitted from the funds provider terminal 30 in response to the funds provider's operation on the browser (S110), the member registration unit 122 of the operator server 10 registers a record to the member DB 132 based on the received member information (S111). The present embodiment assumes that the member information is transmitted from the funds provider terminal 30 in response to the user B's operation on a member registration screen provided by the website provision unit 121, and the member registration unit 122 registers a record to the member DB 132 based on the received member information. The member information includes a name, a gender, a date of birth, etc.

When a download request for the attempt app 70 is transmitted from the funds provider terminal 30 in response to the funds provider's operation on the browser (S112), the website provision unit 121 transmits the attempt app 70 to the funds provider terminal 30 (S113). The user B then installs the downloaded attempt app 70.

The member key generation unit 331 of the funds provider terminal 30 generates a pair of a private key M and a public key M that is unique to each user who has been registered as a member in response to the funds provider's operation on the attempt app 70, and stores the generated private key M as the member private key 71 of the storage unit 34 (S114). The member key generation unit 331 also generates a public address M using the public key M and stores the generated public address M in the public address 72 of the storage unit 34 (S115).

Further, the member key generation unit 331 transmits the generated public address M along with the user identification information to the operator server 10 (S116). In the present embodiment, the member key generation unit 331 uses the user identification information generated using part or all of the member information which is obtained by, for example, prompting the user B to input the same when the attempt app 70 is installed.

Upon receipt of the public address M along with the user identification information from the funds provider terminal 30, the member registration unit 122 of the operator server 10 stores the public address M in the record of the member DB 132 which is specified by the user identification information (S117).

The user A (attempter) also operates the attempter terminal 40 so as to go through the above processes from steps S108 to S117 to complete the member registration, install the attempt app 70 and transmit the public address M of the user A to the operator server 10.

When a fitness club (certifier) also operates the certifier terminal 50 so as to go through the above processes from steps S108 to S109 and certifier information required for certifier registration is transmitted from the certifier terminal 50 (S110'), the certifier registration unit 123 of the operator server 10 registers a record to the certifier DB 133 based on the received certifier information (S111'). In the present embodiment, certifier information is transmitted from the certifier terminal 50 in response to an operation of a person in charge in the fitness club on a certifier registration screen provided by the website provision unit 121 and the certifier registration unit 123 registers a record to the certifier DB 133 based on the received certifier information. The certifier information includes a certifier's name, a certifier's qualification, a location, etc. Then, "ALL," which indicates that the qualification has been verified by the operator, is associated with a designated campaign in the record of the fitness club in the certifier DB 113.

When a download request for the certifier app 80 is transmitted from the certifier terminal 50 in response to the certifier's operation on the browser (S112'), the website provision unit 121 transmits the certifier app 80 to the certifier terminal 50 (S113'). Then, the person in charge of the fitness club installs the downloaded certifier app 80.

The certifier key generation unit 531 of the certifier terminal 50 generates a pair of a private key T and a public key T that is unique to each user who has been registered as a certifier in response to the certifier's operation on the certifier app 80 and stores the generated private key T as the certifier private key 81 of the storage unit 54 (S114'). The certifier key generation unit 531 also generates a public address T using the public key T and stores the generated public address T as the public address 82 of the storage unit 54 (S115').

Further, the certifier key generation unit 531 transmits the generated public address T along with the certifier identification information to the operator server 10 (S116'). In the present embodiment, the certifier key generation unit 531 uses the certifier identification information generated using part or all of the certifier information which is obtained by, for example, prompting the person in charge of the fitness club to input the same when the certifier app 80 is installed.

Upon receipt of the public address T along with the certifier identification information from the certifier terminal 50, the certifier registration unit 123 of the operator server 10 stores the public address T in the record of the certifier DB 133 which is specified by the certifier identification information (S117').

### (Entry Processing)

When the funds provider purchases a product of the beverage manufacturer having a sticker with the encoded code printed thereon and applies the image capturing unit 36 of the funds provider terminal 30 onto the sticker, the code reading unit 332 of the funds provider terminal 30 reads the private key S from an image including the code captured by the image capturing unit 36 and stores the read private key S as the campaign private key 73 (S201), Further, the code reading unit 332 generates a public address S using the public key S derived from the read private key S and transmits the generated public address S and its own public address M to the operator server 10 (S202). In the present embodiment, the code reading unit 332 transmits, to the operator server 10, the generated public address and the public address M of the user B which has been stored as the public address 72.

The entry acceptance unit 126 of the operator server 10 receives, from the funds provider terminal 30, the public address S and the public address M of the user of the funds provider terminal 30, judges whether the received public address S matches with the public address S stored in the campaign DB 131 (S203), and, when matched, the entry acceptance unit 126 stores, as a relevant campaign, the campaign ID associated with the public address S and the participation status "funds provider" in the record having the corresponding public address M in the member DB 132 (S204). Here, it is assumed that the public address received from the fund receiver terminal 30 of the user B matches with the public address S stored in the campaign DB 131, and the campaign ID "001" and the participation status "funds provider" are stored as the relevant campaign in the record corresponding to the public address M of the user B in the member DB.

The participant information transmission unit 127 transmits to the blockchain network 20, as a transaction, the public address M and the public address T, which have been registered to the DB so as to be associated with the campaign of the beverage manufacture, in order to associate the public address M and the public address T with the smart contract of the campaign (S205).

In the present embodiment, in response to the entry acceptance unit 126 receiving the public address M, the participant information transmission unit 127 transmits to the blockchain network 20, as a transaction, the public address M which has been entered to the campaign having the campaign ID "001" and data related to the certifier's public address T for which the campaign ID "001" or "ALL" has been designated as a designated campaign. As a result, the public address M of the user B is stored as the funds provider in the funds provider data sequence [0] and the public address T of the fitness club is stored in the certifier [0] of the funds provider data sequence [0] (S206).

In response to the funds provider's operation on the attempt app 70, the wallet 333 transmits to the blockchain system 20, as a transaction, fund payment data including a beneficiary account and a remittance amount (S207). In the present embodiment, the wallet 333 transmits the public address 72 of the storage unit 34 to the operator server 10, whereby a list of contract accounts of the campaigns for which the relevant user has been registered to the operator server 10 as a funds provider can be obtained. Here, it is assumed that the user B selects a contract account associated with the campaign ID "001" and the wallet 333 signs the fund payment data including the address of the beneficiary account and the remittance amount of "10 ETHER" with the member private key 71 in the storage unit 34, and transmits the fund payment data to the blockchain system 20, as a transaction.

In response to the funds provider's operation on the attempt app 70, the setting transmission unit 334 of the funds provider terminal 30 transmits various types of setting data as a transaction to the blockchain network 20 (S208). In the present embodiment, the setting data includes the public address M of the funds provider, the amount of funds, the public address M of the attempter, the fund distribution setting for the success of the task, the fund distribution setting for the failure of the task, etc. Here, the setting transmission unit 334 signs various types of setting data designated by the user via the input unit 31 with the member private key 71 of the storage unit 34 and transmits to the blockchain network 20 the signed setting data as a transaction whose destination is the contract account associated with the campaign ID "001."

As a result, values are stored in the respective items of the funds provider data sequence [0] in which the public address M of the user B has been stored as the funds provider, as shown in Fig. 8 (S209). Fig. 8(A) shows the content of data which has been set by the attempt app 70 of the funds provider terminal 30, and Fig. 8(B) shows the data structure of the smart contract stored by the transaction transmitted based on the data in Fig. 8(A).

The setting transmission unit 334 transmits the public address M of the attempter designated in the setting data, along with the information related to the campaign, to the operator server 10 (S210). Here, it is assumed that the setting transmission unit 334 transmits the public address M of the user A along with the contract account to the operator server 10.

Upon the receipt of the information related to the campaign and the public address M of the attempter from the funds provider terminal 30, the attempter acceptance unit 128 stores, as the relevant campaign, the campaign ID specified by the information related to the campaign and the participation status "attempter" in the record having the corresponding public address M in the member DB 132 (S211). Here, the attempter acceptance unit 128 receives the contract account and the public address M of the user A from the funds provider terminal 30 and stores the campaign ID "001" specified by the contract account and the participation status "attempter" in a record of the received public address M.

### (Judgment Data Transmission Processing)

In response to the attempter's operation on the attempt app 70, the judgment data transmission unit 431 of the attempter terminal 40 transmits to the blockchain network 20, as a transaction, data that serves as evidence of an activity for achieving a task (S301). In the present embodiment, the judgment data transmission unit 431 transmits the public address 72 of the storage unit 44 to the operator server 10, whereby a list of contract accounts of the campaigns for which the relevant user has been registered to the operator server 10 as an attempter can be obtained. Here, it is assumed that the user A selects a contract account associated with the campaign ID "001" and the judgment data transmission unit 431 signs the data that serves as evidence with the member private key 71 and transmits the signed data to the blockchain network 20, as a transaction.

As stated earlier, in the present embodiment, if the attempter walks 15 days in total during a 30-day period, each day with 10,000 or more steps, the task is considered as having been successfully achieved. In the present embodiment, the data that serves as evidence may include GPS data during walking and the content address that uniquely identifies each content item, such as image data of the measurement results of a body weight and blood pressure, in addition to step count data. The step count data and the GPS data may be obtained from a step count app installed in the attempter terminal 40 or from a pedometer device equipped with a wireless function.

Here, it is assumed that the judgment data transmission unit 431 signs the step count data [0] shown in Fig. 9(a) with the member private key 71 of the storage unit 44, and transmits the signed step count data as a transaction to the blockchain network 20. As shown in Fig. 9(A), initial data transmitted from the attempter terminal 40 to the blockchain network 20 includes "permission or denial of data disclosure" indicating whether the attempter agrees with a disclosure of his/her own data to the sponsor.

After approving the transmitted transaction, the blockchain network 20 stores data in the smart contract (S302). Fig. 9(B) shows the data structure of the smart contract stored by the transmitted transaction. As shown in Fig. 9(B), a "posting date and time" when the transaction was stored in the blockchain and an "accumulated total of days of 10,000-step achievement" counting the accumulated total of days in each of which the attempter has walked 10,000 or more steps, are added on the smart contract end.

As stated earlier, regarding the content in the data that serves as evidence, the judgment data transmission unit 431 generates a content address that uniquely identifies each content, transmits the generated content address as a transaction to the blockchain network 20 in S301, and transmits actual content along with the content address to the operator server 10 (S303). For example, if the user A transmits an image of the measurement results of the body weight and the blood pressure along with the step count data, the judgment data transmission unit 431 transmits, to the blockchain network 20, a hash value generated from the image data so as to be included in the Evidence hash value shown in Fig. 9(A), and transmits the actual image data along with the content address to the operator server 10.

The content receiving unit 129 of the operator server 10 receives the content address and the content from the attempter terminal 40 and stores them in the content DB134 (S304). As a result, the operator server 10 manages the content using the content address.

The judgment data transmission unit 431 may automatically, and in a manner independent of the attempter's operation, transmit the step count data to the blockchain network 20, as a transaction.

When the attempter visits the store, etc. of the certifier and operates the attempt app 70, the attempt app 70 displays a code in which the public address 72 has been encoded on the display unit 42 of the attempter terminal 40 (S305).

In response to the certifier's operation on the certifier app 80, the code reading unit 532 of the certifier terminal 50 reads the public address M from an image including the code which has been captured by the image capturing unit 56 (S306). Here, the code reading unit 532 of the certifier terminal 50 reads the public address M of the user A.

As a result of the attempter's later activity at the store and the certifier's operation on the certifier app 80, the judgment data transmission unit 533 of the certifier terminal 50 may transmit to the blockchain network 20, as a transaction, the data that serves as evidence of the activity for achieving the task (S307). In the present embodiment, the judgment data transmission unit 533 generates data that serves as evidence, such as a store-visit record, based on the public address M read by the code reading unit 532, and signs the generated data with the certifier private key 81 in the storage unit 54 and transmits the signed data to the blockchain network 20.

After approving the transmitted transaction, the blockchain network 20 stores the data in the smart contract (S308).

As stated earlier, regarding the content in the data that serves as evidence, the judgment data transmission unit 533 generates a content address that uniquely identifies each content item, transmits the generated content address as a transaction to the blockchain network 20 in S307, and transmits actual content along with the content address to the operator server 10 (S309).

The content receiving unit 129 of the operator server 10 receives the content address and the content from the certifier terminal 50 and stores them in the content DB 134 (S310).

If the user A achieves the task before the closing date of the smart contract, the smart contract is automatically implemented, so that a refund is distributed in accordance with the fund distribution setting for the success of the task. In the present embodiment, 9.5 ETHER is refunded to the son of the user A, and 0.5 ETHER is remitted to the operator. Further, after the closing date of the smart contract, part of the confiscated money from other attempters who have failed to achieve the task was remitted to the user A.

If the user A cannot achieve the task before the closing date of the smart contract and during the attempt period, the smart contract is automatically implemented, so that a refund is distributed in accordance with the fund distribution setting for the failure of the task. In the present embodiment, 1 ETHER is refunded to the son of the user A, 5 ETHER is refunded to the user B and 1 ETHER is donated to the Japan Red Cross Society. From among the confiscated amount of 3 ETHER, 0.5 ETHER is remitted to the operator, a predetermined amount of compensation is remitted to the certifier, and the remaining confiscated money is equally remitted as rewards to the attempters who have achieved the task.

The judgment data may be transmitted only from the attempter terminal 40, or only from the certifier terminal 50, or from both of them, depending on the contract content of the task. For example, in the task of walking, the judgment data is transmitted only from the attempter terminal 40; in the task of going to a fitness club, the judgment data is transmitted only from the certifier terminal 50; and in the task including both walking and going to a fitness club, the judgment data is transmitted from both of the terminals. However, even in the task for which the judgment data is transmitted only from the certifier terminal 50, it is desirable that the attempter terminal 40 transmits to the blockchain 20, as a transaction, the "permission or denial of data disclosure" data, etc. that indicates whether the attempter agrees with a disclosure of his/her own data to the sponsor by the deadline for starting the attempt, to thereby clearly indicate the start of the attempt.

As described above, according to the present embodiment, it is possible to increase the possibility of an attempter achieving a task by intentionally creating a situation in which, if the task cannot be achieved by a predetermined date and time, the attempter, or those around the attempter, will face an economic cost or let an opportunity to obtain an economic benefit slip away. The user B, who is a funds provider supporting the healthy activity of the user A, can lead the user A to the achievement of the task without directly encouraging the user A. The son of the user A is able to not only attain the enhancement of his father's health but also obtain an economic benefit, by supporting the healthy activity of the user A. According to the present embodiment, the money which an elderly user B has saved without consuming is withdrawn due to the user B's love for his/her family and passed to the user A or the user A's son who are in generations with great consumer confidence, whereby it is possible to expect improvement of the economy.

### (Further Embodiment)

The first embodiment has described an example in which an attempter and a funds provider are different, wherein the attempter is a user A, and the funds provider is a user B who is a father of the user A and who supports a healthy activity of the user A, as shown in the center column of the bottom part of Fig. 13.

Alternatively, the present invention may be applied to an example in which a user C who is an attempter also serves as a funds provider as shown in the left or right column of the bottom part of Fig. 13. In such case, a control unit of a terminal which serves as both the funds provider terminal 30 and the attempter terminal 40 includes a member key generation unit 331, a code reading unit 332, a wallet 333, a setting transmission unit 334 and a judgment data transmission unit 341.

### (Second Embodiment)

A second embodiment will describe an example in which an attempter attempts a task related to household duties. In the second embodiment, if an attempter cleans up after dinner two or more times a week, a task is considered as having been successfully achieved. The second embodiment does not involve an operator, a sponsor or a certifier, and it only involves: a user C, who serves as both a funds provider and an attempter; a user D, being a wife of the user C, desiring to make the user C, who usually does not help out with household duties, actually help out with some of the household duties, as a fund receiver in the case of a failure to achieve the task; and a user E, being a child of the user C who supports a household activity of the user C.

With respect to the user C who has always said that he helps out with some household duties but has not helped out with any household duties so far, the user D signs a contract with an automatic implementation function to the effect that: "using 10 ETHER provided by the user C as funds, if the user C does not clean up after dinner two or more times a week, the user D will be given 10 ETHER; whereas if the user C does clean up after dinner two or more times a week, 8 ETHER will be refunded to the user C and 2 Ether will be given to the user E," and data indicating whether the user C has actually cleaned up is transmitted by the user C.

A control unit of a user C terminal includes a terminal contract generation unit, in addition to the member key generation unit 331, the wallet 333 and the judgment data transmission unit 341.

Since the member key generation unit 331 and the wallet 333 have already been described, the description thereof will be omitted in the following description.

The terminal contract generation unit generates and compiles a code for a smart contract based on a contract condition including a condition for achieving the task and the attempt period, an amount of funds to be deposited for the task, the fund distribution setting for a success of the task, the fund distribution setting for a failure of the task, and a person who judges whether the contract condition has been satisfied, and transmits the compiled code to the blockchain network 20, as a transaction.

More specifically, the terminal contract generation unit generates and compiles a code for a smart contract based on the contract condition, including a condition for achieving the task being "cleaning up after dinner two or more times a week" and a period for attempting the task being "from December 15, 2018 to December 22, 2018," as well as an amount of funds to be deposited for the task being "10 ETHER," the fund distribution setting for the success of the task being "8 ETHER to the user C and 2 ETHER to the user E," the fund distribution setting for the failure of the task being "10 ETHER to the user D," and a person who judges whether the contract condition has been satisfied being the "user C," and transmits the compiled code to the blockchain network 20, as a transaction.

The judgment data transmission unit 431 transmits to the blockchain network 20, as a transaction, the judgment data used for judging whether the contract condition has been satisfied. In the second embodiment, the judgment data transmission unit 431 signs, with the member private key 71 of the storage unit 34, judgment data including either the "success" or "failure" of the task based on a user input through, for example, pushing a "success" button or a "failure" button displayed on a display unit, and transmits the signed judgment data to the blockchain network 20.

As described above, according to the present embodiment, it is possible to increase the possibility of an attempter achieving a task by intentionally creating a situation in which, if the task cannot be achieved by a predetermined date and time, the attempter, or those around the attempter, will face an economic cost or will let an opportunity to obtain an economic benefit slip away. The user D, who desires to make the user C help out with household duties, can lead the user C to the achievement of the task without directly encouraging the user C. The user E, who is a child of the user C, can obtain an economic benefit by supporting the user C helping out with household duties.

The second embodiment has described an example in which the user C serves as the "person who judges whether the contract condition has been satisfied"; however, the present invention is not limited to such embodiment, and the user D or the user E may serve as the "person who judges whether the contract condition has been satisfied," or another arbitrary user may serve as the "person who judges whether the contract condition has been satisfied." If the user D or the user E serves as the "person who judges whether the contract condition has been satisfied," the user D or the user E may possibly transmit judgment data that does not reflect the actual situation. In such case, the user D or the user E would temporarily obtain an economic benefit. However, in such case, the user D or the user E will lose the user C's trust and the user C will never use this system again, which will consequently cause the user D or the user E to lose their chances to continuously obtain an economic benefit in the future. Under such circumstances, it can be considered that the user D and the user E will transmit judgment data based on the actual situation, thinking that they will obtain a larger economic benefit in the long term by making a correct judgment.

The first embodiment and the second embodiment have described examples of using the blockchain network 20 in order to implement the present invention; the present invention is not limited to these embodiments and the present invention can be implemented using a server device having a function corresponding to the blockchain network 20. For example, the present invention may be implemented by using a server device that includes: receiving unit that receives, from a terminal device, a contract condition including a condition for achieving a task and a period for attempting the task, an amount of funds, a fund distribution setting for a success of the task, and a fund distribution setting for a failure of the task, and stores them in a storage unit that is communicably connected to the server device; checking unit that checks a credit of the funds from the terminal device to a predetermined account; judging unit that judges whether the contract condition has been satisfied based on judgment data received from the terminal device; and transferring unit that transfers the funds from the predetermined account to distributed fund receivers based on the fund distribution setting for the success of the task if the contract condition is satisfied during the period, while transferring the funds from the predetermined account to distributed fund receivers based on the fund distribution setting for the failure of the task if the contract condition is not satisfied during the period.

Examples of the transmission of the judgment data from the terminal device to the server device herein include, in addition to the judgment data transmission unit 431 transmitting judgment data to the server device based on a user input through, for example, pushing a "success" button or a "failure" button of a task displayed on a display unit: the terminal device accessing the server device to upload the judgment data; and the terminal device transmitting the judgment data to the server device based on a user input through, for example, pushing a "success" button or a "failure" button of a task displayed on a webpage screen provided by the server device.

Although the first embodiment and the second embodiment have described examples of a task without its deadline, such description is not intended to exclude an application of the present invention to a task with a deadline and, by applying the present invention to a task with a deadline, it is possible to further increase the possibility of the attempter achieving the task.

### (Additional Embodiment)

The operator may generate and manage, in Ethereum, a new token contract related to a health token to be distributed to an attempter him/herself and/or a person supporting the attempter. The token contract is a smart contract having a correspondence table between a plurality of public addresses and the token balances of the respective public addresses. For example, if the attempter achieves a task, the contract generation unit 124 of the operator server 10 may generate a token contract related to a health token of the attempter and remit health tokens issued by the operator, etc., to the relevant token contract, so that the health tokens can be automatically distributed to the respective public addresses in the token contract based on the distribution which has been set by the attempter in accordance with the degrees of contribution of the people who have supported the attempter. The operator server 10 may further include, separately from the contract generation unit 124, a token contract generation unit that transmits contract data related to a health token of the attempter to the blockchain network 20 to thereby generate a token contract related to the health token of the attempter. The token contract generation unit receives, from the blockchain network 20, a contract account issued when the token contract of the health token is issued and stores the received contract account in the member DB 132. The amount of health tokens remitted when the task is successfully achieved may vary depending on the difficulty of the task.

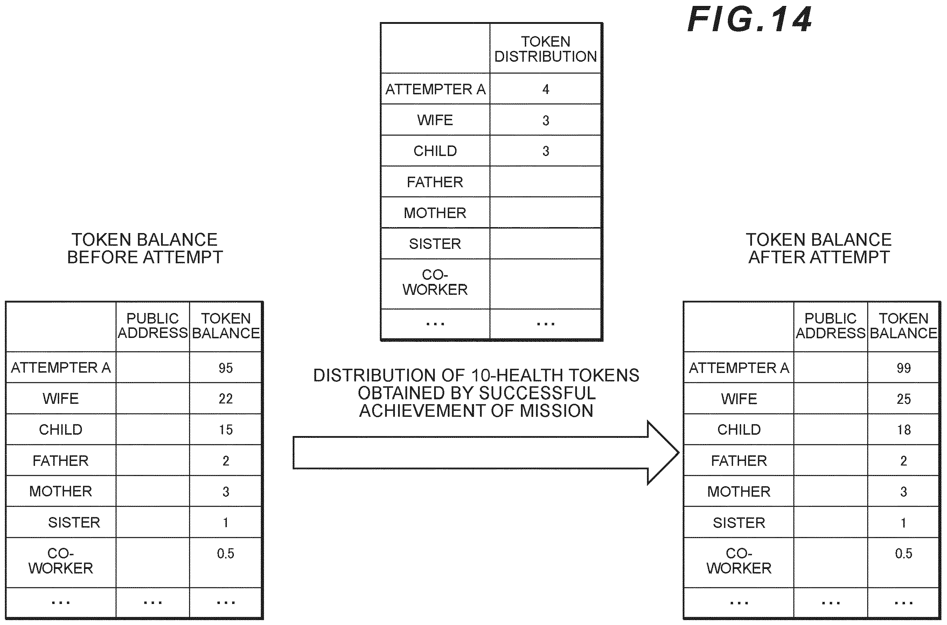

As described earlier, the attempter may distribute the health tokens to be remitted when the task is successfully achieved in accordance with the degree of contribution of the support during the attempt period. Fig. 14 is a conceptual diagram showing a data structure of a token contract of a user A, being an attempter. As shown in Fig. 14, while the attempter continues to attempt a healthy task and achieves the task, people having a higher degree of contribution to the success of the task will get a higher token balance.

By utilizing such token contract, for example, a life insurance company may pay a cashback by ETHER to a token contract of a subscriber who has a large health token balance, and the cashback ETHER may be distributed to the respective public addresses in accordance with the proportions of the health token balances held in the token contract. With such configuration, it is possible to motivate the people who are supporting the healthy activity of the subscriber to actively support the healthy activity of the subscriber.

In another example, a health insurance society may remit part of a health promoting budget to a token contract of a subscriber, so that the remitted ETHER may be distributed to the respective public addresses in accordance with the health token balances held in the token contract. With such configuration, the health insurance society can support people who are supporting the healthy activity of the subscriber.

### Reference Signs List

1: attempt assistance system, 10: operator server, 11: input unit, 12: control unit, 121: website provision unit, 122: member registration unit, 123: certifier registration unit, 124: contract generation unit, 125: key generation unit, 126: entry acceptance unit, 127: participant information transmission unit, 128: attempter acceptance unit, 129: content receiving unit, 13: storage unit, 131: campaign DB, 132: member DB, 133: certifier DB, 134: content DB, 135: private key, 136: public address, 14: communication unit, 20: blockchain network (information processing system), 30: funds provider terminal (participant terminal, terminal device), 31: input unit, 32: display unit, 33: control unit, 331: member key generation unit, 332: code reading unit (entry unit), 333: wallet, 334: setting transmission unit, 34: storage unit, 35: communication unit, 36: image capturing unit, 40: attempter terminal (participant terminal, terminal device), 41: input unit, 42: display unit, 43: control unit, 431: judgment data transmission unit, 44: storage unit, 45: communication unit, 46: image capturing unit, 50: certifier terminal, 51: input unit, 52: display unit, 53: control unit, 531: certifier key generation unit (transmission unit), 532: code reading unit, 533: judgment data transmission unit, 54: storage unit, 55: communication unit, 56: image capturing unit, 60: sponsor server, 70: attempt app, 71: member private key, 72: public address, 73: campaign private key, 80: certifier app, 81: certifier private key, 82: public address, N: network

## Claims

1. An attempt assistance system comprising an operator server, a blockchain network and a plurality of participant terminals,
wherein the operator server comprises:
a contract generation unit that transmits, to the blockchain network, a contract condition related to a task which a user should achieve, the contract condition including a condition for achieving the task and a deadline for achieving the task;
an entry acceptance unit that receives information indicating an entry to the task from the participant terminal; and
a participant information transmission unit that transmits, to the blockchain network, information of a participant whose entry has been accepted, and
wherein the participant terminal comprises:
an entry unit that transmits information indicating an entry to the task to the operator server;
a setting transmission unit that transmits, to the blockchain network, an amount of funds to be deposited for the task, a fund distribution setting for a success of the task and a fund distribution setting for a failure of the task, the fund distribution setting for the success and the fund distribution setting for the failure each including one or more distributed fund receivers' information and an amount of funds to be distributed to each of the distributed fund receivers; and
a judgment data transmission unit that transmits, to the blockchain network, judgment data used for judging whether the contract condition has been satisfied,
wherein a smart contract included in the blockchain network judges whether the contract condition has been satisfied based on the judgment data transmitted from the participant terminal and remits the funds to the respective distributed fund receivers in accordance with the fund distribution setting for the success of the task if the contract condition is satisfied by the deadline, and remits the funds to the respective distributed fund receivers in accordance with the fund distribution setting for the failure of the task if the contract condition is not satisfied by the deadline.

2. The attempt assistance system according to claim 1, wherein:
the operator server further comprises a key generation unit that generates information for participating in the task and stores the generated information in a storage unit that is communicably connected to the operator server; and
the entry acceptance unit receives the information for participating in the task from the participant terminal, and accepts an entry from the participant terminal if the information for participating in the task received from the participant terminal matches with information stored in the storage unit.

3. The challenge assistance system according to claim 1 or 2, wherein:
the operator server further comprises a content receiving unit that receives content and information identifying the content from the judgment data transmission unit of the participant terminal and stores the content and the information identifying the content in a storage unit which is communicably connected to the operator server; and
the judgment data transmission unit of the participant terminal transmits the judgment data including the information identifying the content to the blockchain network and transmits the content and the information identifying the content to the operator server.

4. The attempt assistance system according to any one of claims 1 to 3,
wherein the attempt assistance system further comprises a certifier terminal that comprises:
a transmission unit that transmits information of a certifier to the operator server; and
a judgment data transmission unit that transmits, to the blockchain network, judgment data used for judging whether the contract condition has been satisfied,
wherein the operator server further comprises a certifier registration unit that receives information of the certifier from the certifier terminal, and
wherein the participant information transmission unit transmits the information of the certifier to the blockchain network.

5. The attempt assistance system according to any one of claims 1 to 4,
wherein the participant terminals include a first participant terminal comprising at least the entry unit and the setting transmission unit, and a second participant terminal comprising at least the judgment data transmission unit.

6. The attempt assistance system according to any one of claims 1 to 5,
wherein the operator server further comprises a token contract generation unit that transmits ,to the blockchain network, data related to a token for each participant, and
wherein, if the contract condition has been satisfied by the deadline, the smart contract of the blockchain network transmits a token to a token contract generated in the blockchain network due to the transmission of the data related to the token.

7. A terminal device, comprising:
an entry unit that transmits, to an operator server, information indicating an entry to a task that a user should achieve;
a setting transmission unit that transmits, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers' information and an amount of funds to be distributed to each of the distributed fund receivers; and
a judgment data transmission unit that transmits, to the blockchain network, judgment data used for judging whether a contract condition related to the task has been satisfied.

8. A method, comprising steps performed by a computer, the steps comprising:
transmitting, to an operator server, information indicating an entry to a task that a user should achieve;
transmitting, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers and an amount of funds to be distributed to each of the distributed fund receivers; and
transmitting, to the blockchain network, judgment data used for judging whether a contract condition related to the task has been satisfied.

9. A program causing a computer to perform:
a process of transmitting, to an operator server, information indicating an entry to a task that a user should achieve;
a process of transmitting, to a blockchain network, an amount of funds to be deposited for the task, a fund distribution setting for a success of the task and a fund distribution setting for a failure of the task, the fund distribution setting for the success and the fund distribution setting for the failure each including one or more distributed fund receivers' information and an amount of funds to be distributed to each of the distributed fund receivers; and
a process of transmitting, to the blockchain network, judgment data used for judging whether a contract condition related to the task has been satisfied.

10. A terminal device, comprising:
a terminal contract generation unit that transmits, to a blockchain network, a contract condition including a condition for achieving a task and a period for attempting the task, an amount of funds to be deposited for the task, a fund distribution setting for a success of the task, and a fund distribution setting for a failure of the task; and
a judgment data transmission unit that transmits, to the blockchain network, judgment data used for judging whether the contract condition has been satisfied.

11. The terminal device according to claim 10, wherein:
a plurality of the terminal devices include a first terminal device comprising at least the terminal contract generation unit, and a second terminal device comprising at least the judgment data transmission unit;
the terminal contract generation unit further transmits, to the blockchain network, information of a person who judges whether the contract condition has been satisfied; and
the second terminal device transmits the judgment data along with information associated with the information of the person who judges whether the contract condition has been satisfied.

12. An attempt assistance system, comprising a terminal device and a server device,
wherein the terminal device comprises:
first transmitting unit that transmits, to the server device, a contract condition including a condition for achieving a task and a period for attempting the task, an amount of funds to be deposited for the task, a fund distribution setting for a success of the task, and a fund distribution setting for a failure of the task; and
second transmitting unit that transmits, to the server device, judgment data used for judging whether the contract condition has been satisfied, and
wherein the server device comprises:
receiving unit that receives, from the terminal device, the contract condition, the amount of funds, the fund distribution setting for the success of the task, and the fund distribution setting for the failure of the task, and stores the received information in a storage unit that is communicably connected to the server device;
checking unit that checks a credit of the funds from a user specified by the terminal device to a predetermined account;
judging unit that judges whether the contract condition has been satisfied based on the judgment data received from the terminal device; and
transferring unit that transfers the funds from the predetermined account to distributed fund receivers based on the fund distribution setting for the success of the task if the contract condition is satisfied during the period, while transferring the funds from the predetermined account to distributed fund receivers' account based on the fund distribution setting for the failure of the task if the contract condition is not satisfied during the period.

13. The attempt assistance system according to claim 12, wherein;
the terminal device comprises a first terminal device comprising at least the first transmitting unit, and a second terminal device comprising at least the second transmitting unit;
the first transmitting unit further transmits, to the server device, information of a person who judges whether the contract condition has been satisfied; and
the second terminal device transmits the judgment data along with information associated with the information of the person who judges whether the contract condition has been satisfied.

14. An information processing device, comprising:
first receiving unit that receives, from a first terminal device, a contract condition including a condition for achieving a task and a period for attempting the task, an amount of funds to be deposited for the task, a fund distribution setting for a success of the task, and a fund distribution setting for a failure of the task, and stores the received information in a storage unit;
checking unit that checks a credit of the funds from a user specified by the first terminal device to a predetermined account;
second receiving unit that receives, from a second terminal device, judgment data used for judging whether the contract condition has been satisfied;
judging unit that judges whether the contract condition has been satisfied based on the judgment data; and
transferring unit that transfers the funds from the predetermined account to distributed fund receivers based on the fund distribution setting for the success of the task if the contract condition is satisfied during the period, while transferring the funds from the predetermined account to distributed fund receivers based on the fund distribution setting for the failure of the task if the contract condition is not satisfied during the period.

15. The attempt assistance system according to any one of claims 1 to 6, wherein the fund distribution setting for the failure of the task includes a setting for distributing confiscated money, which is part or all of the funds, to a user different from the user who has failed the task.

16. The attempt assistance system according to claim 15, wherein the user different from the user who has failed the task is a user who has achieved the task from among users who have entered to the task.

17. The attempt assistance system according to claim 16, wherein the confiscated money is equally distributed to users who have successfully achieved the task.

18. The attempt assistance system according to any one of claims 1 to 6, wherein the fund distribution setting for the failure of the task includes a setting for distributing part or all of the funds to an operator of the operator server.

19. An attempt assistance system comprising a terminal device and a server device,
wherein the terminal device comprises:
first transmitting unit that transmits, to the server device, a period for attempting a task and an amount of funds a user deposits for the task; and
second transmitting unit that transmits, to the server device, judgment data used for judging whether a condition for achieving the task has been satisfied during the period, and
wherein the server device comprises:
receiving unit that receives the period and the amount of funds from the terminal device and stores the period and the amount of funds in a storage unit that is connected communicably connected to the server device;
judging unit that judges whether the condition for achieving the task has been satisfied during the period based on the judgment data received from the terminal device; and
confiscating unit that confiscates the funds from the user if it is judged that the condition for achieving the task has not been satisfied during the period.

20. The attempt assistance system according to claim 18, wherein the terminal device comprises a first terminal device comprising at least the first transmitting unit, and a second terminal device comprising at least the second transmitting unit, wherein:
the first transmitting unit further transmits, to the server device, information of a person who judges whether the condition for achieving the task has been satisfied during the period; and
the second terminal device transmits the judgment data along with information associated with the person who judges whether the condition for achieving the task has been satisfied during the period.

21. An information processing device, comprising:
first receiving unit that receives, from a first terminal device, a period for attempting a task and an amount of funds the user deposits for the task, and stores the period and the amount of funds in a storage unit;
second receiving unit that receives, from a second terminal device, judgment data used for judging whether a condition for achieving the task has been satisfied during the period;
judging unit that judges whether the condition for achieving the task has been satisfied during the period based on the judgment data; and
confiscating unit that confiscates the funds from the user if it is judged that the condition for achieving the task has not been satisfied during the period.
